# EUROPEAN PATENT APPLICATION

(11) **EP 2 495 244 A1**
(43) Date of publication of application: **05.09.2012**
(21) Application number: 11156619.6
(22) Date of filing: 02.03.2011
(51) Int. Cl.: C07D 405/14, A61K 31/4709, A61K 31/4725, A61P 29/00, A61P 9/00, A61P 11/00, A61P 25/28, A61P 35/00, A61P 37/00

(54) **Piperidinyl benzoimidazole derivatives as mPGEs-1 inhibitors**

(71) Applicant: NovaSaid AB, 171 65 Solna (SE)
(72) Inventor: Wannberg, Johan, 752 60, UPPSALA (SE); Alterman, Mathias, 741 42, Knivsta (SE); Malm, Johan, 142 66, Trångsund (SE)
(74) Representative: Allee, Harriet Eva Charlotta

(57) **Abstract**

A compound of formula (I): 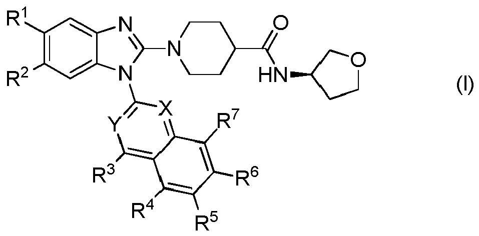 as well as pharmaceutically acceptable salts thereof, and a pharmaceutical composition comprising the compound. The compound is useful for the treatment of disorder selected from inflammatory diseases, pain, auto-immune disease, breathing disorders, fever, cancer, inflammation related anorexia, overactive bladder, familial adenomatous polyposis (FAP) condition, neurodegenerative diseases, bone diseases and cardiovascular diseases.

## Description

### FIELD OF THE INVENTION

The present invention relates to piperidinyl benzoimidazole derivatives and to the use thereof in disease therapy. Particularly, the present invention relates to compounds useful in the treatment or prevention of diseases in which inhibition of the activity of microsomal prostaglandin E synthase-1 is desired and/or required and, more particularly, in the treatment of diseases such as inflammatory diseases, pain, auto-immune disease, breathing disorders, fever, cancer, inflammation-related anorexia, familial adenomatous polyposis, overactive bladder, neurodegenerative and cardiovascular disease.

### BACKGROUND OF THE INVENTION

The following is provided as background information only and represents the current state-of-art in the scientific literature. Furthermore, this background information should not be taken as an admission that any subject matter discussed or that any reference mentioned is prior art to the instant invention.

Prostaglandin (PG) E₂ is produced in a sequential action including liberation of arachidonic acid, conversion into PGG₂/PGH₂ by cyclooxygenase (Cox) -1 or Cox-2 and, finally, conversion of PGH₂ into PGE₂ by prostaglandin E synthase. There exist three known enzymes that catalyze the latter reaction, i.e. microsomal prostaglandin E synthase-1 (mPGEs-1), cytosolic prostaglandin E synthase (cPGEs), and microsomal prostaglandin E synthase-2 (mPGEs-2). The latter two enzymes are constitutively expressed whereas mPGEs-1 is inducible. Initially, mPGEs-1 was regarded as the enzyme predominantly coupled with Cox-2 activity. However; later results demonstrate that mPGEs-1 can also catalyze the conversion of Cox-1 derived PGH₂ into PGE₂. mPGEs-1 possesses the highest catalytic efficiency of the known PGE synthases. The role of PGE₂ as one of the most potent mediators of inflammation together with many *in vitro* reports on the presence of mPGEs-1 in different models, including inflammation, suggest this enzyme to be an attractive drug target for development of new anti inflammatory drugs with fewer side effects than the currently available NSAIDs and selective Cox-2 inhibitors [Samuelsson, B.; Morgenstern, R.; Jakobsson, P.-J. Membrane Prostaglandin E Synthase-1: A Novel Therapeutic Target. Pharmacol. Rev. 2007, 59,207-224; Jachak, S. M. PGE synthase inhibitors as an alternative to COX-2 inhibitors. Curr. Opin. Investig. Drugs, 2007, 8, 411-415]. The rationale is that mPGEs-1 is predominantly expressed during inflammation and that other enzymes exist as mediating "house keeping functions".

Furthermore, mPGEs-1-derived PGE₂ has been implicated in a range of diseases, not confined to inflammation, and it is suggested that inhibitors of mPGEs-1 are effective for the treatment or prevention of these diseases also. Consequently, examples of treatable diseases of the invention include inflammatory diseases, pain, auto-immune diseases, breathing disorders, fever, cancer, inflammation-related anorexia, overactive bladder, familial adenomatous polyposis, neurodegenerative and cardiovascular diseases.

More specifically, non-limiting examples of these diseases include the treatment or prevention of rheumatoid arthritis [Murakami, M.; Nakashima, K.; Kamei, D.; Masuda, S.; Ishikawa, Y.; Ishii, T.; Ohmiya, Y.; Watanabe, K.; Kudo, 1. Cellular prostaglandin E2 production by membrane-bound prostaglandin E synthase-2 via both cyclooxygenases-1 and -2. J. Biol. Chem. 2003, 278, 37937-37947; Westman, M.; Korotkova, M.; af Klint, E.; Stark, A.; Audoly, L. P.; Klareskog, L.; Ulfgren, A. K.; Jakobsson, P. J. Expression of microsomal prostaglandin E synthase 1 in rheumatoid arthritis synovium. Arthritis Rheum. 2004, 50, 1774-1780] or other inflammatory diseases related to arthritis [Kojima, F.; Kato, S.; Kawai, S. Prostaglandin E synthase in the pathophysiology of arthritis. Fundam. Clin. Pharmacol. 2005, 19, 255-261; Fahmi, H. mPGEs-1 as a novel target for arthritis. Curr. Opin. Rheumatol. 2004, 16, 623-627], ankylosing spondylitis [Schett, G.; Rudwaleit, M. Can we stop progression of ankylosing spondylitis? Best Pract Res Clin Rheumatol. 2010, 24, 363-371.], inflammatory bowel disease [Dey, I.; Giembycz, M. A.; Chadee, K. Prostaglandin E(2) couples through EP(4) prostanoid receptors to induce IL-8 production in human colonic epithelial cell lines. Br. J. Pharmacol. 2009, 156, 475-85], osteoarthritis [Gosset, M.; Pigenet, A.; Salvat C.; Berenbaum, F.; Jacques, C. Inhibition of matrix metalloproteinase-3 and -13 synthesis induced by IL-1beta in chondrocytes from mice lacking microsomal prostaglandin E synthase-1. J Immunol, 2010. 185, 6244-52.; Kojima, F.; Naraba, H.; Miyamoto, S.; Beppu, M.; Aoki H, and Kawai S (2004) Membraneassociated prostaglandin E synthase-1 is upregulated by proinflammatory cyto-kines in chondrocytes from patients with osteoarthritis. Arthritis Res. Ther. 2004, R355-R365; Masuko-Hongo, K.; Berenbaum, F.; Humbert, L.; Salvat, C.; Goldring, M. B.; Thirion, S. Up-regulation of microsomal prostaglandin E synthase 1 in osteoarthritic human cartilage: critical roles of the ERK-1/2 and p38 signaling pathways. Arthritis Rheum. 2004, 50, 2829-2838], dermatitis including psoriasis [Schlotter Y.M.; Riemers F.M.; Rutten V.P.; Knol E.F.; Willemse T. Enzymes involved in the conversion of arachidonic acid to eicosanoids in the skin of atopic dogs. Exp. Dermatol., 2010, 8, 317-319; Stark, K.; Törmä, H.; Oliw, E. H. Co-localization of COX-2, CYP4F8, and mPGEs-1 in epidermis with prominent expression of CYP4F8 mRNA in psoriatic lesions. Prostaglandins Other Lipid Mediat. 2006, 79, 114-125] and eczema, swelling, inflammatory pain [Trebino, C. E.; Stock, J. L.; Gibbons, C. P.; Naiman, B. M.; Wachtmann, T. S.; Umland, J. P.; Pandher, K.; Lapointe, J. M.; Saha, S.; Roach, M. L, Carter, D.; Thomas, N. A.; Durtschi, B. A.; McNeish, J. D.; Hambor, J. E.; Jakobsson, P. J.; Carty, T. J.; Perez, J. R.; Audoly, L. P. Impaired inflammatory and pain responses in mice lacking an inducible prostaglandin E synthase. Proc. Natl. Acad. Sci. USA, 2004, 100, 9044-9049], post-operative pain [Shavit, Y.; Fridel, K.; Beilin, B. Postoperative pain management and proinflammatory cytokines: animal and human studies. J. Neuroimmune Pharmacol. 2006, 443-451], fibromyalgia, bone loss [Kwak, H.B.; Sun, H.M.; Ha, H.; Kim, H.N.; Lee, J.H.; Kim, H.H.; Shin, H.I.; Lee, Z.H. Tanshinone IIA suppresses inflammatory bone loss by inhibiting the synthesis of prostaglandin E2 in osteoblasts. Eur J Pharmacol. 2008, 601 ,30-37], periodontitis [Noguchi, K.; Ishikawa, 1. The roles of cyclooxygenase-2 and prostaglandin E2 in periodontal disease. Periodontol. 2000, 2007, 43, 85-101], influenza, dysmenorrhea [Proctor, M.; Farquhar, C. Dysmenorrhoea. Clin. Evid., 2002, 7,1639-1653], migraine, arthrotic and arthritic pain [Schaible, H. G.; Ebersberger, A.; von Banchet, G. S. Mechanisms of pain in arthritis. Ann. N YAcad. Sci. 2002, 966, 343-354], pain in connection with osteoarthritis, cephalalgia (headache) [Wienecke, T.; Olesen, J.; Oturai, P. S.; Ashina, M. Prostaglandin E2(PGE2) induces headache in healthy subjects. Cephalalgia, 2009, 29, 509-519], pain due to gall-stones, pain due to kidney stones, pain due to gout, pain due to metastasis, neuropathic pain [Kunori, S.; Matsumura, S.; Okuda-Ashitaka, E.; Katano, T.; Audoly, L.P.; Urade, Y.; Ito, S. A novel role of prostaglandin E2 in neuropathic pain: blockade of microglial migration in the spinal cord. Glia, 2011, 59, 208-218], familial adenomatous polyposis (FAP) condition [Takeda, H.; Sonoshita, M.; Oshima, H.; Sugihara, K.; Chulada, P. C.; Langenbach, R.; Oshima, M.; Taketo, M. M. Cooperation of cyclooxygenase 1 and cyclooxygenase 2 in intestinal polyposis. Cancer Res. 2003, 63, 4872-4877], endometriosis [Lousse, J.C.; Defrère, S.; Colette, S.; Van Langendonckt, A.; Donnez, J. Expression of eicosanoid biosynthetic and catabolic enzymes in peritoneal endometriosis. Hum. Reprod., 2010, 25, 734-741], intestinal tumorogenesis [Nakanishi, M.; Montrose, D.C.; Clark, P.; Nambiar, P.R.; Belinsky, G.S.; Claffey, K.P.; Xu, D.; Rosenberg, D.W. Genetic deletion of mPGES-1 suppresses intestinal tumorigenesis. Cancer Res., 2008, 68, 3251-3259.], colorectal cancer [Talero, E.; Sánchez-Fidalgo, S.; Villegas, I.; de la Lastra, C. A.; Illanes, M.; Motilva, V. Role of different inflammatory and tumor biomarkers in the development of ulcerative colitis-associated carcinogenesis. Inflamm Bowel Dis. 2010, Aug 18.; Kamei, D.; Murakami, M.; Nakatani, Y.; Ishikawa, Y.; Ishii, T.; Kudo, I. Potential role of microsomal prostaglandin E synthase-1 in tumorigenesis. J. Biol. Chem., 2003, 278, 19396-19405; Yoshimatsu, K.; Golijanin, D.; Paty, P. B.; Soslow, R. A.; Jakobsson, P.-J.; DeLellis, R. A.; Subbaramaiah, K.; Dannenberg, A. J. Inducible microsomal prostaglandin E synthase is overexpressed in colorectal adenomas and cancer. Clin. Cancer Res. 2001, 7, 3971-3976; Elander, N.; Ungerbäck, J.; Olsson, H.; Uematsu, S.; Akira, S.; Soderkvist, P. Genetic deletion of mPGEs-1 accelerates intestinal tumorigenesis in APC(Min/+) mice. Biochem. Biophys. Res. Commun. 2008, 372, 249-253], epithelial ovarian cancer [Rask, K.; Zhu, Y.; Wang, W.; Hedin, L.; Sundfeldt, K. Ovarian epithelial cancer: a role for PGE2-synthesis and signalling in malignant transformation and progression. Mol. Cancer, 2006, 16, 62], breast cancer [Mehrotra, S.; Morimiya, A.; Agarwal, B.; Konger, R.; Badve, S. Microsomal prostaglandin E2 synthase-1 in breast cancer: a potential target for therapy. J. Pathol. 2006, 208, 356-363], gastric tumorigenesis [Oshima, H.; Matsunaga, A.; Fujimura, T.; Tsukamoto, T.; Taketo, M. M.; Oshima, M. Carcinogenesis in mouse stomach by simultaneous activation of the Wnt signaling and prostaglandin E2 pathway. Gastroenterology, 2006, 131, 1086-1095], bone metastatic cancer [Inada, M.; Miyaura, C. Role of PGE2 in bone metastatic cancer. Clin. Calcium. 2008, 18, 466-472], lung cancer [Wu, Y.C.; Su, L.J.; Wang, H.W.; Jeff Lin C.F.; Hsu, W.H.; Chou, T.Y.; Huang, C.Y.; Lu, C.L.; Hsueh, C.T. Co-overexpression of cyclooxygenase-2 and microsomal prostaglandin E synthase-1 adversely affects the postoperative survival in non-small cell lung cancer. J. Thorac. Oncol. 2010, 8, 1167-1174.], oesophageal adenocarcinoma [Beales, I.L.; Ogunwobi, O.O. Microsomal prostaglandin E synthase-1 inhibition blocks proliferation and enhances apoptosis in oesophageal adenocarcinoma cells without affecting endothelial prostacyclin production. Int. J. Cancer, 2010, 126, 2247-2255], Head and neck squamous cell carcinoma, Papillary thyroid carcinoma, Gliomas and pancreatic, cervical and prostate cancers [reviewed in: Nakanishi, M.; Gokhale, V.; Meuillet, EJ.; Rosenberg, D.W. mPGES-1 as a target for cancer suppression: A comprehensive invited review "Phospholipase A2 and lipid mediators". Biochimie. 2010, 92, 660-664; Yip-Schneider, M. T.; Wu, H.; Njoku, V.; Ralstin, M.; Holcomb, B.; Crooks, P. A.; Neelakantan, S.; Sweeney, C. J.; Schmidt, C. M. Effect of celecoxib and the novel anti-cancer agent, dimethylamino-parthenolide, in a developmental model of pancreatic cancer. Pancreas 2008, 37(3), e45-53], multiple sclerosis [Kihara, Y.; Matsushita, T.; Kita, Y.; Uematsu, S.; Akira, S.; Kira, J.; Ishii, S.; Shimizu, T. Targeted lipidomics reveals mPGES-1-PGE2 as a therapeutic target for multiple sclerosis. Proc Natl Acad Sci U S A., 2009, 106, 21807-21812], seizure-induced neuronal damage [Takemiya, T.; Matsumura, K.; Sugiura, H.; Yasuda, S.; Uematsu, S.; Akira, S.; Yamagata, K. Endothelial microsomal prostaglandin Esynthase-1 facilitates neurotoxicity by elevating astrocytic Ca2+ levels, Neurochemistry International,2010;Takemiya, T., Matsumura, K., Sugiura, H., Maehara, M., Yasuda, S., Uematsu, S., Akira, S. and amagata, K., Endothelial microsomal prostaglandin E synthase-1 exacerbates neuronal loss induced by kainate. J Neurosci.Res. 2010. 88, 381-390] atherosclerosis, myocardial infarction and stroke [Wang, M.; Song, W.L.; Cheng, Y.; Fitzgerald, G.A. Microsomal prostaglandin E synthase-1 inhibition in cardiovascular inflammatory disease. J Intern Med., 2008, 263, 500-505. Ikeda-Matsuo Y.; Hirayama Y.; Ota A.; Uematsu S., Akira S., Sasaki Y., Microsomal prostaglandin E synthase-1 and cyclooxygenase-2 are both required for ischaemic excitotoxicity. Br. J. Pharmacol. 2010, 159, 1174-1186; Ikeda-Matsuo, Y.; Ota, A.; Fukada, T.; Uematsu, S.; Akira, S.; Sasaki, Y. Microsomal prostaglandin E synthase-1 is a critical factor of stroke-reperfusion injury. Proc. Natl. Acad. Sci. USA. 2006, 103, 11790-11795], apnea or other respiratory distress symptoms in adults and children [Hofstetter, A. O.; Saha, S.; Siljehav, V.; Jakobsson, P. J.; Herlenius, E. The induced prostaglandin E2 pathway is a key regulator of the respiratory response to infection and hypoxia in neonates. Proc. Natl. Acad. Sci. USA. 2007, 104, 9894-9899], sudden infant death syndrome (SIDS), asthma, fever [Engblom, D.; Saha, S.; Engström, L.; Westman, M.; Audoly, L. P.; Jakobsson, P. J.; Blomqvist, A. Microsomal prostaglandin E synthase-1 is the central switch during immune-induced pyresis. Nat. Neurosci. 2003, 6, 1137-1138; Saha, S.; Engström, L.; Mackerlova, L.; Jakobsson, P. J.; Blomqvist, A. Impaired febrile responses to immune challenge in mice deficient in microsomal prostaglandin E synthase-1. Am. J. Physiol. 2005, 288, R1100-R1107], inflammation-related anorexia [Samuelsson, B.; Morgenstern, R.; Jakobsson, P.-J. Membrane Prostaglandin E Synthase-1: A Novel Therapeutic Target. Pharmacol. Rev. 2007, 59,207-224], amyotrophic lateral sclerosis [Liang, X.; Wang, Q.; Shi, J.; Lokteva, L.; Breyer, R.M.; Montine, T.J.; Andreasson, K. The prostaglandin E2 EP2 receptor accelerates disease progression and inflammation in a model of amyotrophic lateral sclerosis. Ann Neurol. 2008, 64, 304-314.], Alzheimer's disease [Satoh, K.; Nagano, Y.; Shimomura, C.; Suzuki, N.; Saeki, Y.; Yokota, H. Expression of prostaglandin E synthase mRNA is induced in beta-amyloid treated rat astrocytes. Neurosci. Lett. 2000, 283, 221-223; Licastro, F.; Davis, L.J.; Pedrini, S.; Galasko, D.; Masliah, E. Prostaglandin E2 induced polymerization of human alpha-1-antichymotrypsin and suppressed its protease inhibitory activity: implications for Alzheimer's disease. Biochem. Biophys. Res. Commun. 1998, 249, 182-186; Chaudhry, U. A.; Zhuang, H.; Crain, B. J.; Doré, S. Elevated microsomal prostaglandin-E synthase-1 in Alzheimer's disease. Alzheimers Dement. 2008, 4, 6-13; Hoozemans, J. J.; Veerhuis, R.; Janssen, I.; van Elk, E. J.; Rozemuller, A. J.; Eikelenboom, P. The role of cyclooxygenase 1 and 2 activity in prostaglandin E(2) secretion by cultured human adult microglia: implications for Alzheimer's disease. Brain Res. 2002, 951, 218-226; Scali, C.; Prosperi, C.; Bracco, L.; Piccini, C.; Baronti, R.; Ginestroni, A.; Sorbi, S.; Pepeu, G.; Casamenti, F. Neutrophils CD11b and fibroblasts PGE(2) are elevated in Alzheimer's disease. Neurobiol. Aging, 2002, 23, 523-30], memory impairment [Matousek, S.B.; Hein, A.M.; Shaftel, S.S.; Olschowka, J.A.; Kyrkanides, S.; O'Banion, M.K. Cyclooxygenase-1 mediates prostaglandin E(2) elevation and contextual memory impairment in a model of sustained hippocampal interleukin-1beta expression. J Neurochem., 2010, 114, 247-258.], overactive bladder, pachydermoperiostosis, Kawasaki disease, inflammation-related nephrototoxicity [Jia, Z.; Wang, N.; Aoyagi, T.; Wang, H.; Liu, H.; Yang, T. Amelioration of cisplatin nephrotoxicity by genetic or pharmacologic blockade of prostaglandin synthesis. Kidney Int. 2011, 79, 77-88], (intra-) ocular inflammation [Yanni, S. E.; Barnett, J. M.; Clark, M. L.; Penn, J. S. The role of PGE2 receptor EP4 in pathologic ocular angiogenesis. Invest. Ophthalmol. Vis. Sci. 2009, 50, 5479-5486; Barañano, D. E.; Kim, S. J.; Edelhauser, H. F.; Durairaj, C.; Kompella, U. B.; Handa, J. T. Efficacy and pharmacokinetics of intravitreal non-steroidal anti-inflammatory drugs for intraocular inflammation. Br. J Ophthalmol. 2009, 93, 1387-1390] as well as the use of the said compounds in the manufacture of medicaments for the treatment of those disorders [For a reviews discussing most of the therapeutic indications mentioned above see: Samuelsson, B.; Morgenstern, R.; Jakobsson, P.-J. Membrane Prostaglandin E Synthase-1: A Novel Therapeutic Target. Pharmacol. Rev. 2007, 59,207-224; Murakami M, Kudo I. Prostaglandin E synthase: a novel drug target for inflammation and cancer. Curr Pharm Des. 2006, 12, 943-954; Kojima, F.; Kato, S.; Kawai, S. Prostaglandin E synthase in the pathophysiology of arthritis. Fundam. Clin. Pharmacol. 2005, 19, 255-261; Rådmark, O; Samuelsson, B. Microsomal prostaglandin E synthase-1 and 5-lipoxygenase: potential drug targets in cancer. J. Intern. Med. 2010, 268, 5-14.]

WO/2007/042816 and WO/2008/071944 disclose benzoxazole derivatives of the general formula:

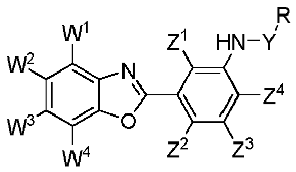

WO 2008/084218 discloses benzazole derivatives of the general formula: 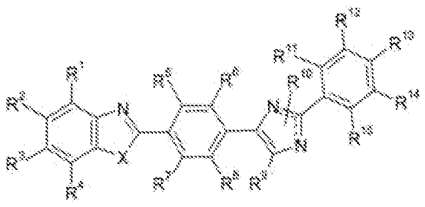 and WO/2010/034797 and WO/2010/034796 disclose benzoimidazoles of the general formula: 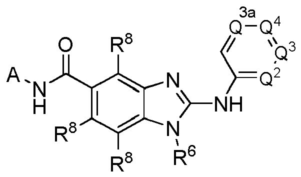 as useful in the treatment of diseases in which it is desired and/or required to inhibit the activity of a member of the MAPEG family, e.g. microsomal prostaglandin E syntliase-1 (mPGEs-1).

### SUMMARY OF THE INVENTION

There still is a large unmet need for drugs that are as effective as NSAID inhibitors but having improved properties, such as increased solubility, higher affinity and/or selectivity, higher efficacy or improved side effect profile, for the treatment of conditions such as inflammatory diseases, pain, auto-immune diseases, breathing disorders, fever, cancer, inflammation related anorexia, treatment and/or prevention of overactive bladder, neurodegenerative diseases and cardiovascular diseases. One object of the present invention is to provide compounds having at least one or more of these desired improved properties.

The present inventors have surprisingly found that certain substituted 1-(1H-benzimidazol-2-yl)piperidine-4-carboxamide derivatives have particularly advantageous mPGEs-1 inhibiting properties that make them very useful in therapy. More specifically, the inventors have found that the presence of an (iso)quinoline moiety, attached to the available benzimidazole nitrogen in said derivatives, has an advantageous effect on the inhibiting activity. Moreover, the inventors have found that the position of the nitrogen in the quinoline or isoquinoline moiety, relative to the benzimidazole ring, seems to have an important effect on enzymatic mPGEs-1 inhibition.

Consequently, in one aspect the present invention provides a compound of formula (I) 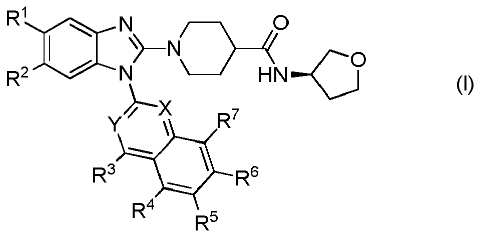 wherein
R¹ and R² are each independently selected from methyl and chlorine;
R³, R⁴, R⁵, R⁶ and R⁷ are each independently selected from hydrogen, fluorine, chlorine, and C₁-C₄ alkyl;
X and Y are both selected from -N- and -CH-, provided that X and Y are different,
or a pharmaceutically acceptable salt thereof.

Another aspect of the present invention is to provide a compound of formula (I) or a pharmaceutically acceptable salt thereof for use as a medicament.

Another aspect of the present invention is to provide a pharmaceutical composition comprising a therapeutically effective amount of a compound of formula (I), or a pharmaceutically acceptable salt thereof, in admixture with at least one pharmaceutically acceptable excipient, e.g. an adjuvant, diluent or carrier.

Another aspect of the present invention is to provide a method of medical treatment by use of said pharmaceutical compositions.

Another aspect of the present invention provides a compound of formula (I) or a pharmaceutically acceptable salt thereof, as defined herein above, for use in the treatment of a disorder in which inhibition of the activity of microsomal prostaglandin E synthase-1 is desired and/or required.

Another aspect of the present invention provides the use of a compound of formula (I), in the preparation of a medicament for the treatment and/or prevention of a disorder in which inhibition of the activity of microsomal prostaglandin E synthase-1 is desired and/or required.

Another aspect of the present invention provides a compound of formula (I), for use in the treatment and/or prevention of an inflammatory disease, said disease preferably being selected from rheumatoid arthritis and other inflammatory diseases related to arthritis such as ankylosing spondylitis, inflammatory bowel disease, inflammation-related nephrototoxicity osteoarthritis, periodontitis, dermatitis including psoriasis, eczema, swelling and (intra-)ocular inflammation.

Another aspect of the present invention provides a compound of formula (I), for use in the treatment and/or prevention of pain, said pain preferably being selected from inflammatory pain, post-operative pain, fibromyalgia, dysmenorrhea, migraine, arthrotic pain, arthritic pain, pain in connection with osteoarthritis, endometriosis, cephalalgia, pain due to gall-stones, pain due to kidney stones, pain due to gout, neuropathic pain and pain due to metastasis.

Another aspect of the present invention provides a compound of formula (I), for use in the treatment and/or prevention of an autoimmune disease, said autoimmune disease preferably being selected from rheumatoid arthritis, multiple sclerosis and Kawasaki disease.

Another aspect of the present invention provides a compound of formula (I), for use in the treatment and/or prevention of a breathing disorder, said disorder preferably being selected from apnea or other respiratory distress symptoms in adults and children, sudden infant death syndrome (SIDS), asthma and other chronic obstructive lung-diseases and sarcoidosis.

Another aspect of the present invention provides a compound of formula (I), for use in the treatment and/or prevention of cancer, said cancer preferably being selected from colorectal cancer, breast cancer, gastric tumorigenesis, intestinal tumorogenesis, bone metastatic cancer, lung cancer, oesophageal adenocarcinoma, head and neck squamous cell carcinoma, papillary thyroid carcinoma, gliomas and pancreatic, cervical and prostate cancers, epithelial ovarian cancer and pancreas cancer.

Another aspect of the present invention provides a compound of formula (I), for use in the treatment and/or prevention of cardiovascular diseases due to atherosclerosis such as myocardial infarction and stroke.

Another aspect of the present invention provides a compound of formula (I), for use in the treatment and/or prevention of overactive bladder, familial adenomatous polyposis (FAP) condition, fever and inflammation-related anorexia.

Another aspect of the present invention provides a compound of formula (I), for use in the treatment and/or prevention of neurodegenerative diseases, said disorder preferably being selected from Alzheimer's disease, amyotrophic lateral sclerosis, memory impairment and seizure-induced neuronal damage.

Another aspect of the present invention provides a compound of formula (I), for use in the treatment and/or prevention of bone diseases, said disorder preferably being selected from bone loss and pachydermoperiostosis (PDP, also known as "primary hypertrophic osteoathropathy").

Another aspect of the present invention provides the use of a compound of formula (I), in the preparation of a medicament for the treatment and/or prevention of an inflammatory disease, said disease preferably being selected from rheumatoid arthritis and other inflammatory diseases related to arthritis such as ankylosing spondylitis , inflammatory bowel disease, inflammation-related nephrototoxicity osteoarthritis, periodontitis, dermatitis including psoriasis, eczema, swelling and (intra-)ocular inflammation.

Another aspect of the present invention provides the use of a compound of formula (I), in the preparation of a medicament for the treatment and/or prevention of pain, said pain preferably being selected from inflammatory pain, post-operative pain, fibromyalgia, dysmenorrhea, migraine, arthrotic pain, arthritic pain, pain in connection with osteoarthritis, endometriosis, cephalalgia, pain due to gall-stones, pain due to kidney stones, pain due to gout, neuropathic pain and pain due to metastasis.

Another aspect of the present invention provides the use of a compound of formula (I), in the preparation of a medicament for the treatment and/or prevention of an autoimmune disease, said autoimmune disease preferably being selected from rheumatoid arthritis, multiple sclerosis and Kawasaki disease.

Another aspect of the present invention provides the use of a compound of formula (I), in the preparation of a medicament for the treatment and/or prevention of a breathing disorder, said disorder preferably being selected from apnea or other respiratory distress symptoms in adults and children, sudden infant death syndrome (SIDS), asthma and other chronic obstructive lung-diseases and sarcoidosis.

Another aspect of the present invention provides the use of a compound of formula (I), in the preparation of a medicament for the treatment and/or prevention of cancer, said cancer preferably being selected from colorectal cancer, breast cancer, gastric tumorigenesis, intestinal tumorogenesis, bone metastatic cancer, lung cancer, oesophageal adenocarcinoma, head and neck squamous cell carcinoma, papillary thyroid carcinoma, gliomas and pancreatic, cervical and prostate cancers, epithelial ovarian cancer and pancreas cancer.

Another aspect of the present invention provides the use of a compound of formula (I), in the preparation of a medicament for the treatment and/or prevention of cardiovascular diseases due to atherosclerosis such as myocardial infarction and stroke.

Another aspect of the present invention provides the use of a compound of formula (I), in preparation of a medicament for the treatment and/or prevention of overactive bladder, familial adenomatous polyposis (FAP) condition, fever and inflammation-related anorexia.

Another aspect of the present invention provides the use of a compound of formula (I), in the preparation of a medicament for the treatment and/or prevention of neurodegenerative diseases, said disorder preferably being selected from Alzheimer's disease, amyotrophic lateral sclerosis, memory impairment and seizure-induced neuronal damage.

Another aspect of the present invention provides the use of a compound of formula (I), in the preparation of a medicament for the treatment and/or prevention of bone diseases, said disorder preferably being selected from bone loss and pachydermoperiostosis (PDP, also known as "primary hypertrophic osteoathropathy").

Another aspect of the present invention provides a method of treatment of a disease in which inhibition of the activity of mPGEs-1 is desired and/or required, which method comprises administration of a therapeutically effective amount of a compound of formula (I), or a pharmaceutically-acceptable salt thereof, to a patient suffering from, or susceptible to, such a condition.

Another aspect of the present invention provides a method of treatment of an inflammatory disease, said disease preferably being selected from rheumatoid arthritis and other inflammatory diseases related to arthritis such as ankylosing spondylitis , inflammatory bowel disease, inflammation-related nephrototoxicity osteoarthritis, periodontitis, dermatitis including psoriasis, eczema, swelling and (intra-)ocular inflammation, which method comprises administration of a therapeutically effective amount of a compound of formula (I), or a pharmaceutically-acceptable salt thereof, to a patient in the need of such treatment.

Another aspect of the present invention provides a method of treatment of pain, said pain preferably being selected from inflammatory pain, post-operative pain, fibromyalgia, dysmenorrhea, migraine, arthrotic pain, arthritic pain, pain in connection with osteoarthritis, endometriosis, cephalalgia, pain due to gall-stones, pain due to kidney stones, pain due to gout, neuropathic pain and pain due to metastasis, which method comprises administration of a therapeutically effective amount of a compound of formula (I), or a pharmaceutically-acceptable salt thereof, to a patient in the need of such treatment.

Another aspect of the present invention provides a method of treatment of an autoimmune disease, said autoimmune disease preferably being selected from rheumatoid arthritis, multiple sclerosis and Kawasaki disease, which method comprises administration of a therapeutically effective amount of a compound of formula (I), or a pharmaceutically-acceptable salt thereof, to a patient in the need of such treatment.

Another aspect of the present invention provides a method of treatment of a breathing disorder, said disorder preferably being selected from apnea or other respiratory distress symptoms in adults and children, sudden infant death syndrome (SIDS), asthma and other chronic obstructive lung-diseases and sarcoidosis, which method comprises administration of a therapeutically effective amount of a compound of formula (I), or a pharmaceutically-acceptable salt thereof, to a patient in the need of such treatment.

Another aspect of the present invention provides a method of treatment of cancer, said cancer preferably being selected from colorectal cancer, breast cancer, gastric tumorigenesis, intestinal tumorogenesis, bone metastatic cancer, lung cancer, oesophageal adenocarcinoma, head and neck squamous cell carcinoma, papillary thyroid carcinoma, gliomas and pancreatic, cervical and prostate cancers, epithelial ovarian cancer and pancreas cancer, which method comprises administration of a therapeutically effective amount of a compound of formula (I), or a pharmaceutically-acceptable salt thereof, to a patient in the need of such treatment.

Another aspect of the present invention provides a method of treatment of cardiovascular diseases, said cardiovascular disease preferable being selected from atherosclerosis such as myocardial infarction and stroke, which method comprises administration of a therapeutically effective amount of a compound of formula (I), or a pharmaceutically-acceptable salt thereof, to a patient in the need of such treatment.

Another aspect of the present invention provides a method of treatment of overactive bladder, familial adenomatous polyposis (FAP) condition, fever and inflammation-related anorexia, which method comprises administration of a therapeutically effective amount of a compound of formula (I), or a pharmaceutically-acceptable salt thereof, to a patient in the need of such treatment.

Another aspect of the present invention provides a method of treatment of neurodegenerative diseases, said neurodegenerative disease preferable being selected from Alzheimer's disease, amyotrophic lateral sclerosis, memory impairment and seizure-induced neuronal damage, which method comprises administration of a therapeutically effective amount of a compound of formula (I), or a pharmaceutically-acceptable salt thereof, to a patient in the need of such treatment.

Another aspect of the present invention provides a method of treatment of bone diseases, said disorder preferably being selected from bone loss and pachydermoperiostosis (PDP, also known as "primary hypertrophic osteoathropathy"), which method comprises administration of a therapeutically effective amount of a compound of formula (I), or a pharmaceutically-acceptable salt thereof, to a patient in the need of such treatment.

In another aspect of the invention, methods for preparing a compound of formula (I) are provided.

### DETAILED DESCRIPTION OF THE INVENTION

Unless otherwise indicated or apparent from the context, any alkyl group as referred to herein may be branched or unbranched.

The term "alkyl" as employed herein, alone or as part of another group, refers to an acyclic straight or branched chain radical, unless otherwise specified containing 1, 2, 3 or 4 carbons in the normal chain, whic*h* includes methyl, ethyl, *n-*propyl and *n-*butyl. Examples of branched *chain radicals are iso*-propyl, *sec*-butyl, *tert*-butyl.

As used herein, the term a "haloheteroaryl" means a heteroaryl, substituted with at least one halogen.

Any chiral center in a compound of formula (I) having a specified configuration is indicated as *R* or *S* using the well-known Cahn-Ingold-Prelog priority rules. Also, in formula (I), in any chiral center having a specified configuration, (i.e. R or S) this is indicated using 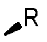 to indicate that the bond to R is directed out of the paper and towards the reader, and 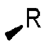 to indicate that the bond to R is directed out of the paper and away from the reader.

In a first aspect of the invention, there is provided a compound of formula (I): 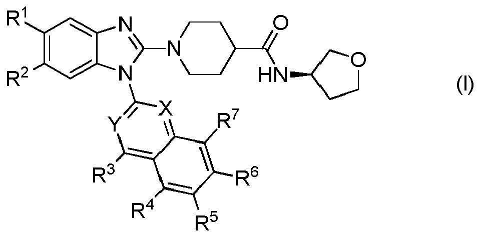 as defined herein above; or a pharmaceutically acceptable salt thereof.

In formula (I), R¹ is selected from methyl and chlorine. In some embodiments, R¹ is chlorine. In some other embodiments, R¹ is methyl.

In formula (I), R² is selected from methyl and chlorine. In some embodiments, R² is chlorine. In some other embodiments, R² is methyl.

In some embodiments, R¹ and R² are both chlorine.

In one embodiment, R¹ and R² are both methyl.

In one embodiment R¹ is methyl and R² is chlorine.

In one embodiment R¹ is chlorine and R² is methyl.

In formula (I), R³, R⁴, R⁵, R⁶ and R⁷ are each independently selected from hydrogen, fluorine, chlorine, and C₁-C₄ alkyl, e.g. from hydrogen, fluorine, chlorine, and methyl.

In some embodiments, R³ is selected from hydrogen and C₁-C₄ alkyl, e.g. hydrogen and methyl, in particular hydrogen.

In some embodiments, R⁴ is selected from hydrogen and fluorine, e.g. from hydrogen.

R⁵ is selected from hydrogen, fluorine, chlorine, and C₁-C₄ alkyl, e.g. from hydrogen, fluorine, chlorine and methyl. In some embodiments, R⁵ is selected from fluorine, chlorine, and C₁-C₄ alkyl, e.g. from fluorine, chlorine and methyl. In some embodiments, R5 is hydrogen.

In some embodiments, R⁵ is selected from hydrogen, fluorine and chlorine, e.g. from fluorine and chlorine.

In some embodiments, R⁶ is selected from hydrogen and fluorine, e.g. from hydrogen.

In some embodiments R⁷ is selected from hydrogen, fluorine and chlorine. In some embodiments, R⁷ is selected from hydrogen and fluorine, in particular from hydrogen. In some embodiments, R⁷ is selected from fluorine and chlorine.

In some embodiments, R⁴ and R⁶ are both hydrogen. In others, R³, R⁴ and R⁶ are hydrogen. In still others, R⁴, R⁶ and R⁷ are hydrogen, or R³, R⁴, R⁶ and R⁷ are hydrogen. In some embodiments, R³, R⁴, R⁵, R⁶ and R⁷ are all hydrogen.

In a compound of formula (I), either X is -N- and Y is -CH-, or X is -CH- and Y is -N-. Preferably X is -N- and Y is -CH-.

In one embodiment of the invention, there is provided a compound selected from:
(*R*)-1-(5,6-dichloro-1-(quinolin-2-yl)-1*H*-benzo[*d*]imidazol-2-yl)-N-(tetrahydrofuran-3-yl)piperidine-4-carboxamide;
(*R*)-1-(5-chloro-6-methyl-1-(quinolin-2-yl)-1H-benzo-[*d*]imidazol-2-yl)-N-(tetrahydrofuran-3-yl)piperidine-4-carboxamide;
(*R*)-1-(6-chloro-5-methyl-1-(quinolin-2-yl)-1*H*-benzo[*d*]imidazol-2-yl)-*N-*(tetrahydrofuran-3-yl)piperidine-4-carboxamide;
(*R*)-1-(5,6-dichloro-1-(6-chloroquinolin-2-yl)-1*H*-benzo[*d*]imidazol-2-yl)-*N*-(tetrahydrofuran-3-yl)piperidine-4-carboxamide;
(*R*)-1-(1-(6-chloroquinolin-2-yl)-5,6-dimethyl-1*H*-benzo[*d*]imidazol-2-yl)-*N*-(tetrahydrofuran-3-yl)piperidine-4-carboxamide;
(*R*)-1-(5,6-dichloro-1-(4-methylquinolin-2-yl)-1*H*-benzo[*d*]imidazol-2-yl)-*N*-(tetrahydrofuran-3-yl)piperidine-4-carboxamide;
(*R)*-1-(5-chloro-1-(6-chloroquinolin-2-yl)-6-methyl-1*H*-benzo[*d*]imidazol-2-yl)-*N-*(tetrahydrofuran-3-yl)piperidine-4-carboxamide;
(*R*)-1-(6-chloro-1-(6-chloroquinolin-2-yl)-5-methyl-1*H*-benzo[*d*]imidazol-2-yl)-*N-*(tetrahydrofuran-3-yl)piperidine-4-carboxamide;
(*R*)-1-(5,6-dichloro-1-(6-fluoroquinolin-2-yl)-1*H*-benzo[*d*]imidazol-2-yl)-*N-*(tetrahydrofuran-3-yl)piperidine-4-carboxamide;
(*R*)-1-(5-chloro-1-(6-fluoroquinolin-2-yl)-6-methyl-1*H*-benzo[*d*]imidazol-2-yl)-*N-*(tetrahydrofuran-3-yl)piperidine-4-carboxamide;
(*R)*-1-(6-chloro-1-(6-fluoroquinolin-2-yl)-5-methyl-1*H*-benzo[*d*]imidazol-2-yl)-*N-*(tetrahydrofuran-3-yl)piperidine-4-carboxamide;
(*R*)-1-(5,6-dichloro-1-(8-fluoroquinolin-2-yl)-1*H*-benzo[*d*]imidazol-2-yl)-*N*-(tetrahydrofuran-3-yl)piperidine-4-carboxamide; and
(*R*)-1-(5,6-dichloro-1-(isoquinolin-3-yl)-1H-benzo[d]imidazol-2-yl)-N-(tetrahydrofuran-3-yl)piperidine-4-carboxamide;
   or a pharmaceutically acceptable salt thereof.

The compounds of formula (I) of the invention can be prepared according to the synthetic routes outlined herein and by following the methods described herein below and illustrated in the Examples. It should be realized, though, that alternative synthetic routes to the inventive compounds can easily be visualized by any person skilled in the art and the present synthetic routes are not limiting for the invention.

With respect to the reaction schemes below, although the various R¹, R², R³, R⁴, R⁵, R⁶, R⁷, X and Y moieties sometimes are specifically defined, unless otherwise indicated, it is to be understood that R¹, R², R³, R⁴, R⁵, R⁶, R⁷, X and Y may be any of the groups encompassed thereby.

As a non-limiting example, a compound of formula (I) as defined herein may be formed in a five step procedure wherein, first, a suitable substituted 2-aminobenzoimidazol (i) is diazotizated and subjected to the Sandmeyer reaction, or any suitable modification of this method, the formed chloro intermediate (ii) coupled with a piperidine-4-carboxylic acid ester (iii) to form the carboxylic acid ester (iv) that is saponified to give the carboxylic acid derivative (v), which subsequently is coupled with tetrahydrofuran-3-amine (vi), and finally *N*-alkylated with a bromoquinoline derivative (viii) bromide to yield the final compound of formula (I).

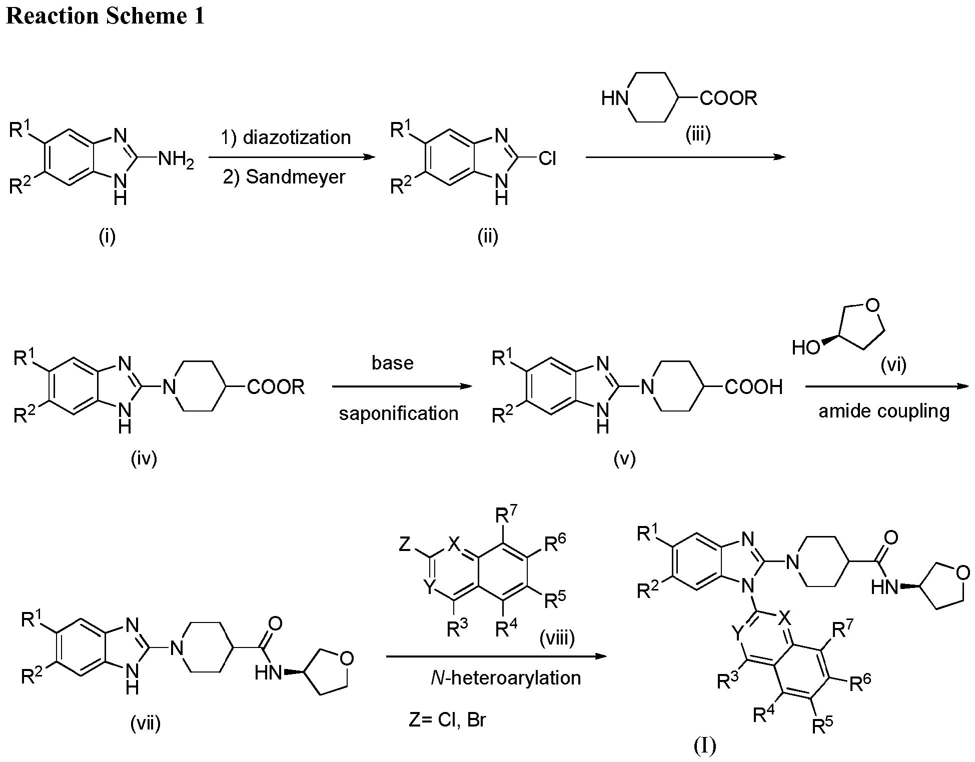

The process of forming diazonium compounds from aromatic or heteroaromatic amines has been known for more than 150 years and involves an amine that reacts with a nitrite salt such as sodium nitrite to form a diazonium salt at low temperature, a procedure that should be well known for the one skilled in the art. Typically diazonium compounds are not isolated, but used immediately in further reactions as described below in the Sandmeyer reaction. Diazonium compounds can, however, also be isolated as tetrafluoroborate salts. Conditions suitable for the Sandmeyer reaction are well known to the person skilled in the art and comprise a diazonium salt of an aromatic or heterocyclic compound kept at low temperature, which is decomposed in the presence of copper(I) salts, such as copper(I) chloride or copper(I) bromide, to form the desired chloro or bromo derivative. Several modifications to the standard procedures above have been made and are exemplified by the use of t-butyl nitrite and anhydrous copper(II) chloride or copper(II) bromide at elevated temperatures [Doyle, M. P.; Siegfried, B.; Dellaria Jr, J. F. Alkyl nitrite-metal halide deamination reactions. 2. Substitutive deamination of arylamines by alkyl nitrites and copper(II) halides. A direct and remarkably efficient conversion of arylamines to aryl halides. J. Org. Chem. 1977, 42, 2426-2431], t-butyl thionitrite and copper(II) chloride or copper(II) bromide employed at room temperature [Oae, A.; Shinhama, K.; Kim, Y. H. Direct conversion of arylamines to the corresponding halides, biphenyls and sulfides with t-butyl thionitrate. Chem. Lett., 1979, 939-942] and mixtures of Cu(I) and Cu(II) chloro and bromo salts, used in catalytic amounts in the presence of the bidentate ligand phenanthroline and phase-transfer catalyst dibenzo-18-crown-6 [Beletskaya, I, P.; Sigeev, A. S.; Peregudov, A. S.; Petrovskii, P. V. Catalytic Sandmeyer Bromination. Synthesis, 2007, 2534-2538].

In this context, it should be realized that a wide variety of methods exist for the transformation of the amine (i) into the chloro compound (ii) and several exhaustive review articles on the topic can be found in the literature.

Conditions suitable for the coupling of aromatic and heteroaromatic halides, such as chlorides, with aliphatic amine chains or secondary amine aliphatic ring structures such as piperidine derivatives as the piperidine-4-carboxylic acid ester (iii) are well known for the one skilled in the art, and there exists a wealth of different methods in the scientific literature, summarized in for example: *"*Advanced Organic Chemistry", 4th edition, Jerry March, Wiley-Interscience publication, 1992, p 656-657, including the references cited therein. In the present invention the chloro intermediate (ii) is reacted with a piperidine-4-carboxylic acid ester derivative (iii) to form the coupled product (iv) in the presence of a base, such as as N,N-diisopropylethylamine (Hünig's base, DIEA, CAS Registry Number: 7087-68-5), in a suitable solvent, such as *iso*propylalcohol, 1,4-dioxane, and the like. Obviously other alternative good bases that are poor nucleophiles can be employed, such as, but not limited to 2,2,6,6-tetramethylpiperidine (TMP, CAS Registry Number: 768-66-1).

The carboxylic acid ester (iv) is saponified with a base such as aqueous sodium hydroxide and a solvent such as ethanol. Acidification of the product is followed by standard work-up, well known to the one skilled in the art, to yield the carboxylic acid (v). Several other bases suitable for saponification exist, such as lithium hydroxide that can be combined with several different solvents. Furthermore, a wide variety of other protecting groups for the carboxylic acid can be employed, and their usage is known to those skilled in the art (references describing protecting group strategy include, for example, *"*Protecting Groups in Organic Chemistry", J. F. W. McOmie, Plenum Press, London, New York, 1973, and *"*Protective Groups in Organic Synthesis", T. W. Greene, Wiley, New York, 1984).

Conditions suitable for amide coupling, as exemplified herein by the carboxylic acid derivative (v) and an amine, such as (R)-3-aminotetrahydrofurane (vi), are well-known to the person of ordinary skill in the art, and may be e.g. obtained by performing the reaction of acid and amine in the presence of a suitable amide coupling reagent, such as 2-(7-aza-*1H*-benzotriazole-1-yl)-1,1,3,3-tetramethyl-uronium hexafluorophosphate (HATU; CAS Registry Number: 148893-10-1) and *N,N*-diisopropylethylamine in a suitable solvent, such as *N,N*-dimethylformamide. Numerous reagents and reaction conditions exist in the scientific literature and are all well known for the one skilled in the art.

In the transformation of the benzimidazole (vii) into the final compound of formula (I), N-heteroarylation by a haloquinoline or haloisoquinoline (viii) is employed. Consequently, the benzimidazole (vii) is treated with (viii) in the presence of a base such as caesium carbonate, and an agent such as 1,10-phenanthroline, 4,7-dimethoxy-1,10-phenanthroline or 8-hydroxyquinoline, polyethylene glycol and a copper species such as copper(I) oxide or copper(I) halide, in a solvent such as dimethylsulfoxide. Yet other combinations of bases, catalysts, solvents and agents can be appropriately employed and are well known for those skilled in the art [e.g. Florian Monnier, F.; Taillefer, M. Catalytic C-C, C-N, and C-O Ullmann-Type Coupling Reactions. Angew. Chem. Int. Ed. 2009, 48, 6954 - 6971], and are all well within the scope of the invention.

Yet other combinations of bases, catalysts, solvents and agents can be appropriately employed and are well known for those skilled in the art [e.g. Preston, P. N. Synthesis, reactions, and spectroscopic properties of benzimidazoles. Chem. Rev., 1974, 74, 279-314], and are all well within the scope of the invention.

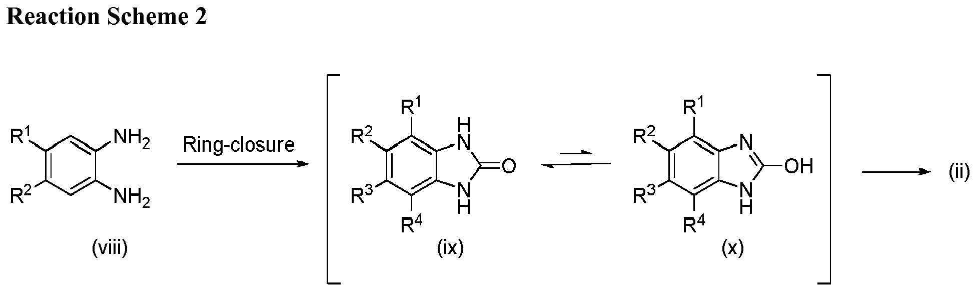

An alternative preparation of (ii) (cf. Reaction Scheme 1), is depicted in Reaction Scheme 2 above and involves the treatment of a suitable substituted diamine (viii) with excess amounts of the carbonyl source *N,N*-carbonyldiimidazole (CDI, CAS Registry Number: 530-62-1) in a solvent such as tetrahydrofuran, in order to achieve ring-closure to the corresponding tautomerically most stable -one form (ix). The ring-closure of aromatic or heteroaromatic diamines is well-known in the literature and might involve the use of alternative carbonyl sources exemplified by the usage of carbon monoxide in the presence of catalytic amounts of tungsten hexacarbonyl (CAS Registry Number: 14040-11-0). Subsequently, the tautomeric -ol form (x) can be transformed to the intermediary (ii) via treatment with phosphoryl trichloride (CAS Registry Number: 10025-87-3), as exemplified in the invention. Alternative reagents include phosphorus pentachloride (CAS Registry Number: 10026-13-8) or phosphorus trichloride (CAS Registry Number: 7719-12-2), the former also as mixtures in various ratios, or any other chlorination agent, of which the scientific literature contains abundant examples, for the purpose of transforming a hydroxyl group to a chlorine group. In this context, it should be realized that also the corresponding bromo derivative of (ii) can be useful as an intermediate in the reaction schemes, leading to the target compounds of the invention, and could be prepared by the action of phosphoryl bromide (CAS Registry Number: 7789-59-5) phosphorus tribromide (CAS Registry Number: 7789-60-8) and/or phosphorus pentabromide (CAS Registry Number: 7789-69-7), or any other brominating agent, abundantly exemplified in the scientific literature, for the purpose of transforming a hydroxyl group to a bromine group.

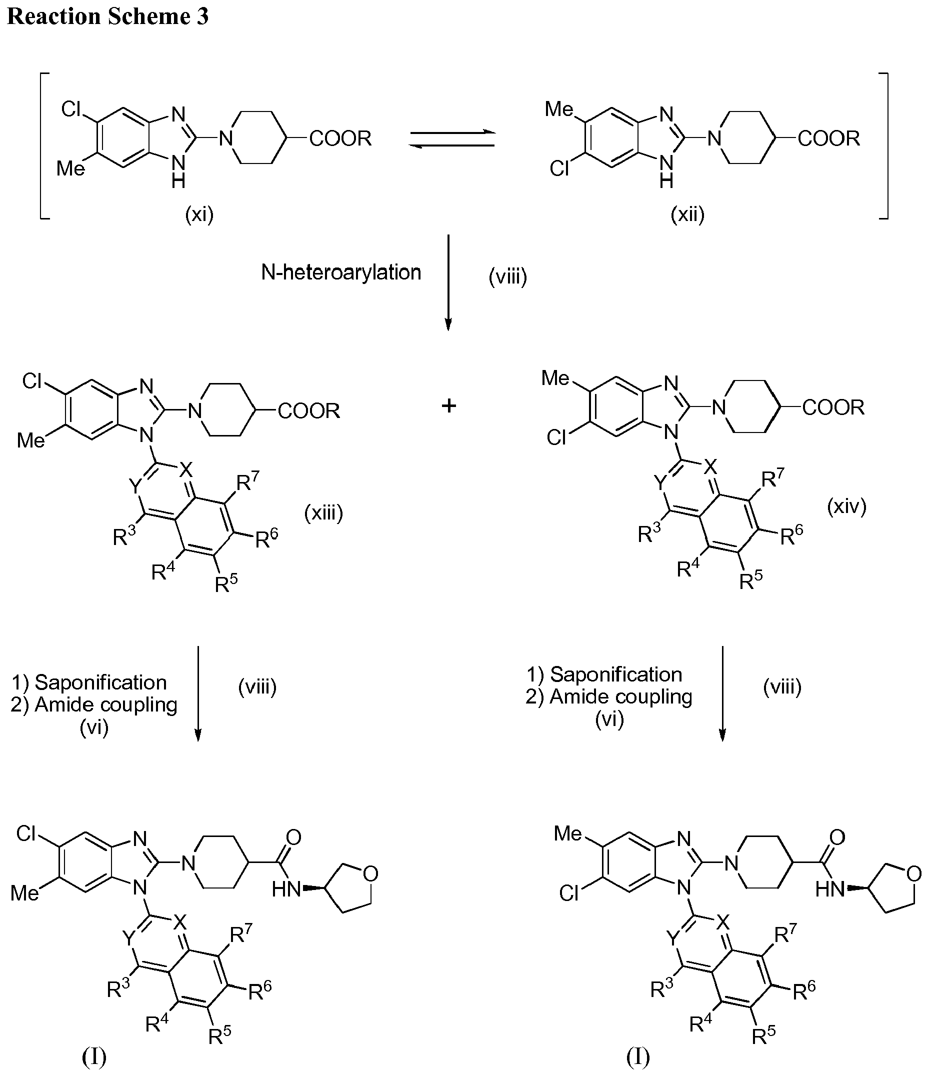

The invention at hand also comprises a synthetic method that involves regioselective control when R¹ and R² are dissimilar. This is outlined in Reaction Scheme 3 and the synthetic strategy involves the introduction of the N-heteroarylating agent (viii) at an early stage in the synthesis in order to obtain control over regioselectivity. This will provide the N-substituted compounds (xiii) and (xiv), which already at this stage can be separated using any of the techniques mentioned above. This synthetic method will, after saponification and amide coupling with (vi), lead to the end products (I) of the invention.

A number of alternative chemical transformations may also be used within the present invention, which may all readily be conducted by the one skilled in the art.

Apart from traditional heating, such as by the use of heating blocks, heat transfer via oil baths or the like, the present invention also involves the use of microwave irridation for heating purposes, which today is well known for the one skilled in the art, as witnessed by a large number of book chapters and published books [e.g. Strauss, C. R., Application of microwaves for environmentally benign organic chemistry, In Handbook of Green Chemistry and Technology, Clark, J.; Macquarrie, D., Eds. Blackwell Science Ltd: Oxford, 2002, pp 297-415; Kappe, C. O.; Stadler, A. Microwaves in Organic and Medicinal Chemistry, Wiley-VCH, Weinheim, 2005; Watley, B.; Tierney, J.; Lidström, P.; Westman, J. The impact of microwave assisted organic chemistry on drug discovery. Drug Discov. Today, 2002, 7, 373-380].

All reaction mixtures are purified by standard purification procedures, such as extraction, washing, recrystallization, and column chromatography employing silica gel, reverse phase and the like, employing the appropriate eluents for the purification of the Examples described herein.

The compounds of the invention can also be prepared by the application of combinatorical and/or parallel chemistry techniques e.g. methods of making libraries of compounds, techniques well known for the one skilled in the art, which can include solution phase synthesis, and solid phase synthesis including design of resins, linkers and the like.

The present invention also comprises stereoisomers that, for example, are prepared with the available metods described above. A large number of available single enantiomers are available commercially or in the literature.

When R¹ and R² are dissimilar, e.g. R¹ is chlorine and R² is methyl, then regioisomers might be formed upon N-heteroarylation with e.g. bromoquinolines (viii). In such case, several spectroscopic methods are available for the structural determination of regioisomers. For example, when R¹ is dissimilar to R², the Nuclear Overhauser effect (NOE) can be utlilized to determine the exact structure of the compound, since the NOE is observed through space, and not through bonds. Consequently, all atoms that are in proximity to each other give a NOE. Thus, the NMR technique NOESY (Nuclear Overhauser Effect SpectroscopY) can be used for determination of the nature of R¹ and R². For example, a NOE effect can be seen between the H^{a}, H^{b} and H^{c} protons in (xi), and reveals that H^{b} correlates with both H^{a} and H^{c} in the 2-dimensional plot. On the other hand, in (xii) H^{a} and H^{d} correlate as a pair, as H^{b} and H^{c}.

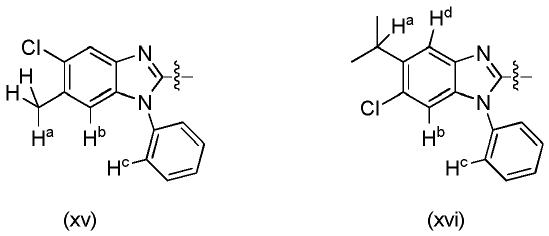

The compounds according to formula (I) will be useful for treating various diseases such as inflammatory diseases, pain, auto-immune disease, breathing disorders, fever, cancer, inflammation-related anorexia, familial adenomatous polyposis, overactive bladder, neurodegenerative and cardiovascular disease. The treatment may be preventive, palliative or curative.

Examples of pharmaceutically acceptable addition salts for use in the pharmaceutical compositions of the present invention include those derived from mineral acids, such as hydrochloric, hydrobromic, phosphoric, metaphosphoric, nitric and sulphuric acids, and organic acids, such as tartaric, acetic, citric, malic, lactic, fumaric, benzoic, glycolic, gluconic, succinic, and arylsulphonic acids. The pharmaceutically acceptable excipients described herein, for example, vehicles, adjuvants, carriers or diluents, are well-known to those who are skilled in the art and are readily available to the public. The pharmaceutically acceptable carrier may be one that is chemically inert to the active compounds and that has no detrimental side effects or toxicity under the conditions of use. Pharmaceutical formulations are found e.g. in Remington: The Science and Practice of Pharmacy, 19th ed., Mack Printing Company, Easton, Pennsylvania (1995).

Except, when otherwise indicated, e.g. by indication of (R) or (S) configuration at a given location, all stereoisomers of the compounds of the instant invention are contemplated, either in admixture or in pure or substantially pure form. The compounds of the present invention can have asymmetric centers at any of the carbon atoms including any one of the substitutents (R¹-R⁷). Consequently, compounds of formula (I) can exist in enantiomeric or diasteromeric forms or in mixtures thereof. The processes for preparation can utilize racemates, enantiomers or diasteromers as starting materials. When diastereomeric or enantiomeric products are prepared, they can be separated by conventional methods, which for example are chromatographic or fractional crystallization.

Prodrugs of the compounds of formula (I) may be prepared by modifying functional groups present on the compound in such a way that the modifications are cleaved, in vivo when such prodrug is administered to a mammalian subject. The modifications typically are achieved by synthesizing the parent compound with a prodrug substituent. Prodrugs include compounds of formula (I) wherein a hydroxy, amino, sulfhydryl or carbonyl group in a compound of formula (I) is bonded to any group that may be *cleaved in vivo* to regenerate the free hydroxyl, amino, or sulfhydryl group, respectively. Examples of prodrugs include, but are not limited to, esters and carbamates of hydroxy functional groups, esters groups of carboxyl functional groups, N-acyl derivatives, N-Mannich bases. General information on prodrugs may be found in the scientific literature [e.g. Bundegaard, H. "Design of Prodrugs" p 1-92, Elesevier, New York-Oxford, 1985; The Practice of Medicinal Chemistry, Camille G. Wermuth et al., chapter 31, Academic Press, 1996; A Textbook of Drug Design and Development, P. Krogsgaard-Larson and H. Bundgaard, eds. chapter 5, pgs 113 - 191, Harwood Academic Publishers, 1991). Said references are incorporated herein by reference. All prodrugs of the compounds of the invention are included within the scope of the invention.

The compounds of the formula (I) can be administered for any of the uses described herein by any suitable means, for example, orally, such as in the form of tablets, aqueous or oily suspensions or solutions, elixirs, syrups, capsules, granules or powders; sublingually; buccally; ocularly; parenterally, such as by transdermal, subcutaneous, intravenous, intramuscular, or intrasternal injection or infusion techniques (e.g., as sterile injectable aqueous or non-aqueous solutions or suspensions). Also, the compounds of the formula (I) may be applied as gargles and mouth washes. For parenteral administration, a parenterally acceptable aqueous or oily suspension, emulsion or solution is employed, which is pyrogen free and has requisite pH, isotonicity, osmolality and stability. Those skilled in the art are well able to prepare suitable formulations and numerous methods are described in the literature. A brief review of methods of drug delivery is also found in the scientific literature [e.g. Langer, Science 249:1527-1533 (1990)]. Furthermore, the compounds of the formula (I) can be administered nasally, including administration to the nasal membranes, such as by inhalation spray; topically, such as in the form of a gel, cream or ointment; or rectally such as in the form of suppositories; in dosage unit formulations containing non-toxic, pharmaceutically acceptable vehicles or diluents. The present compounds can, for example, be administered in a form suitable for immediate release or extended release. Immediate release or extended release can be achieved by the use of suitable pharmaceutical compositions comprising the present compounds, or, particularly in the case of extended release, by the use of devices such as subcutaneous implants or osmotic pumps. The present compounds can also be administered liposomally. The precise nature of the carrier or other material will depend on the route of administration and those skilled in the art are well able to prepare suitable solutions and numerous methods are described in the literature.

Exemplary compositions for oral administration include suspensions which can contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which can contain, for example, microcrystalline cellulose, dicalcium phosphate, starch, magnesium stearate and/or lactose and/or other excipients, binders, extenders, disintegrants, diluents and lubricants such as those known in the art. The compounds of formula (I) can also be delivered through the oral cavity by sublingual and/or buccal administration. Molded tablets, compressed tablets or freeze-dried tablets are exemplary forms which may be used. Exemplary compositions include those formulating the present compound(s) with fast dissolving diluents such as mannitol, lactose, sucrose and/or cyclodextrins. Also included in such formulations may be high molecular weight excipients such as celluloses (avicel) or polyethylene glycols (PEG). Such formulations can also include an excipient to aid mucosal adhesion such as hydroxy propyl cellulose (HPC), hydroxy propyl methyl cellulose (HPMC), sodium carboxy methyl cellulose (SCMC), maleic anhydride copolymer (e.g., Gantrez), and agents to control release such as polyacrylic copolymer (e.g. Carbopol 934). Lubricants, glidants, flavors, coloring agents and stabilizers may also be added for ease of fabrication and use.

Exemplary compositions for nasal aerosol or inhalation administration include solutions in saline which can contain, for example, benzyl alcohol or other suitable preservatives, absorption promoters to enhance bioavailability, and/or other solubilizing or dispersing agents such as those known in the art.

Exemplary compositions for parenteral administration include injectable solutions, emulsions or suspensions which can contain, for example, suitable non-toxic, parenterally acceptable diluents or solvents, such as mannitol, 1,3-butanediol, water, Ringer's solution, an isotonic sodium chloride solution, oil or other suitable dispersing or wetting and suspending agents, including synthetic mono- or diglycerides, and fatty acids, including oleic acid, or Cremaphor.

Exemplary compositions for rectal administration include suppositories which can contain, for example, a suitable non-irritating excipient, such as cocoa butter, synthetic glyceride esters or polyethylene glycols, which are solid at ordinary temperatures, but liquify and/or dissolve in the rectal cavity to release the drug.

Exemplary compositions for topical administration include a topical carrier such as Plastibase (mineral oil gelled with polyethylene).

The dose administered to a mammal, particularly a human, in the context of the present invention should be sufficient to effect a therapeutic response in the mammal over a reasonable time frame. One skilled in the art will recognize that dosage will depend upon a variety of factors including the potency of the specific compound, the age, condition and body weight of the patient, the nature and extent of the condition being treated, recommendations of the treating physician, and the therapeutics or combination of therapeutics selected for administration, as well as the stage and severity of the disease. The dose will also be determined by the route (administration form), timing and frequency of administration. Oral dosages of the present invention, when used for the indicated effects, will range between about 0.01 mg per kg of body weight per day (mg/kg/day) to about 100 mg/kg/day, preferably 0.01 mg per kg of body weight per day (mg/kg/day) to 20 mg/kg/day, and most preferably 0.1 to 10 mg/kg/day, for adult humans. For oral administration, the compositions are preferably provided in the form of tablets or other forms of presentation provided in discrete units containing 0.5 to 1000 milligrams of the active ingredient for the symptomatic adjustment of the dosage to the patient to be treated, for example 0.5, 1.0, 2.5, 5.0, 10.0, 15.0, 25.0, 50.0, 100, 200, 400, 500, 600 and 800 mg.

Parenterally, especially intravenously, the most preferred doses will range from about 0.001 to about 10 mg/kg/hour during a constant rate infusion. Advantageously, compounds of the present invention may be administered in a single daily dose, or the total daily dosage may be administered in divided doses of two, three or four times daily. Furthermore, preferred compounds for the present invention can be administered in intranasal form via topical use of suitable intranasal vehicles, or via transdermal routes, using those forms of transdermal skin patches well known to those of ordinary skill in the art, or using so-called active transdermal technologies such as iontophoresis, electroporation, microneedles, abrasion, needle-less injection etc.

To be administered in the form of a transdermal delivery system, the dosage administration will, of course, be continuous rather than intermittent throughout the dosage regimen. A nasal, sublingual or buccal administration typically contains from about 0.01 mg to about 500 mg of the active ingredient, preferably from about 1 mg to about 100 mg of active ingredient, per dose.

The compounds of the present invention may also be used or administered in combination with one or more additional drugs useful in the treatment of inflammatory diseases, pain, auto-immune diseases, breathing disorders, fever, cancer, inflammation related anorexia, Alzheimer's disease and cardiovascular diseases. The components may be in the same formulation or in separate formulations for administration simultaneously or sequentially. The compounds of the present invention may also be used or administered in combination with other treatment such as irradiation for the treatment of cancer.

Accordingly, in a further aspect of the invention, there is provided a combination product comprising:
(A) a compound of the invention, as hereinbefore defined; and
(B) another therapeutic agent that is useful in the treatment of inflammatory diseases, pain, auto-immune disease, breathing disorders, fever, cancer, inflammation related anorexia, Alzheimer's disease and cardiovascular diseases, wherein each of components (A) and (B) is formulated in admixture with a pharmaceutically acceptable excipient.

Such combination products provide for the administration of a compound of the invention in conjunction with the other therapeutic agent, and may thus be presented either as separate formulations, wherein at least one of those formulations comprises a compound of the invention, and at least one comprises the other therapeutic agent, or may be presented (i.e. formulated) as a combined preparation (i.e. presented as a single formulation including a compound of the invention and the other therapeutic agent).

Thus, there is further provided:
(1) a pharmaceutical formulation including a compound of the invention, as hereinbefore defined, another therapeutic agent, and a pharmaceutically acceptable excipient, e.g. an adjuvant, diluent or carrier; and
(2) a kit of parts comprising components:
   (a) a pharmaceutical formulation including a compound of the invention, as hereinbefore defined, in admixture with a pharmaceutically acceptable excipient, e.g. an adjuvant, diluent or carrier; and
   (b) a pharmaceutical formulation including another therapeutic agent in admixture with a pharmaceutically acceptable excipient, e.g. an adjuvant, diluent or carrier, which components (a) and (b) are each provided in a form that is suitable for administration in conjunction with the other.

Compounds of the formula (I) will be useful as partial or complete substitute for cyclooxygenase-2 inhibitors and conventional NSAIDs in preparations wherein they are presently co-administered with other agents or ingredients. Examples of such agents for the administration in combination with compounds of the invention, include suitable anti-inflammatory, anti-pain, anti-autoimmune, anti-fever, anti-cancer and anti-anorexia (inflammatoriy) agents, and agents for the treatment or prevention of breathing disorders, cardiovascular diseases and Alzheimer's disease. a potentiator including caffeine, an H2 antagonist, aluminium or magnesium hydroxide, simethicone, a decongestant, an antitussive, an antihistamine, a diuretic, a proton pump inhibitor, a bradykinin-1 antagonist, a sodium channel blocker, a 5-HT agonist or a CGRP antagonist. More specifically compounds for use in combination with the compounds of the present invention include, but are not limited to, prednisone (CAS Registry Number: 53-03-2); dexamethasone (CAS Registry Number: 50-02-2); any of the selective glucocorticoid receptor agonists (SEGRAs) exemplified by A276575 (Lin, C. et al. Mol. Pharm., 2002, 62, 297-303), and ZK209614 (Schäcke, H. et al, Proc. Natl. Acad. Sci. USA., 2004,101, 227-232); aspirin (CAS Registry Number: 50-78-2); indomethacin (CAS Registry Number: 53-86-1) particularly dosed as local treatment with gel or spray; ibuprofen (CAS Registry Number: 15687-27-1); piroxicam (CAS Registry Number: 36322-90-4,; CTLA4-Ig agonists/antagonists (Kremer, J. M. et al, NEngl J Med. 2003, 349, 1907-1915)*;* Inosine-5'-monophosphate dehydrogenase inhibitors (Whitby, F. G. et al., Biochemistry, 1997, 36, 10666-10674), such as mycophenolate (CAS Registry Number: 24280-93-1); tumor necrosis factor (TNF) antagonists as infliximab (CAS Registry Number: 170277-31-3); adalimumab (CAS Registry Number: 331731-18-1); etanercept (CAS Registry Number: 185243-69-0) and pentoxifylline (CAS Registry Number: 6493-05-6); orazipone (OR-1384); integrin antagonists; cell adhesion inhibitors;interferon gamma antagonists; budesonide (CAS Registry Number: 51333-22-3); clofazimine (CAS Registry Number: 2030-63-9); selective thyroid hormone agonists such as GC-1 (Johansson, L. et al.,Proc. Natl. Acad. Sci. USA. 2005, 102, 10297-10302), KB2115 (Berkenstam, A. et al., Proc. Natl. Acad. Sci. USA. 2008,105, 663-667); KB-141 (Grover, G. J. et al., Proc. Natl. Acad. Sci. USA. 2003, 100, 10067-10072)*;* and MB07811 (Erion, MD et al., Proc. Natl. Acad. Sci. USA. 2007, 104, 15490-15495); selective farnesoid X receptor agonists (FXRs) such as WAY-362450 (Flatt, B. et al., J. Med. Chem. 2009, 52, 904-907); CD4 antagonists such as priliximab (CAS Registry Number: 147191-91-1); p38 mitogen-activated protein kinase inhibitors such as SB203580 (CAS Registry Number: 152121-47-6); mesalazine (CAS Registry Number: 89-57-6); azathioprine (CAS Registry Number: 446-86-6); sumatriptan (CAS Registry Number: 103628-46-2); paracetamol (CAS Registry Number: 103-90-2); fast-acting bronchodilators such as salbutamol (CAS Registry Number: 18559-94-9) and ephedrine (CAS Registry Number: 299-42-3); rituximab; NO-releasing drugs such as nitroglycerine (CAS Registry Number: 55-63-0) and isosorbide dinitrate (CAS Registry Number: 87-33-2); Selective Estrogen Receptor Modulators (SERMs) such as tamoxifen (CAS Registry Number: 10540-29-1); raloxifene (CAS Registry Number: 84449-90-1) and toremifene (CAS Registry Number: 89778-26-7); selective Liver X receptor (LXR) agonists such as T0901317 (CAS Registry Number: 293754-55-9, Repa, J. J. et al., Science, 2000, 289, 1524-1529) and GW3965 (CAS Registry Number: 405911-17-3); anti-depressant drugs and pain-relivers, such as tricyclic antidepressants (TCAs), selective serotonin reuptake inhibitors (SSRIs) and serotoninnorepinephrine reuptake inhibitors (SNRIs) such as desvenlafaxine (CAS Registry Number: 93413-62-8); duloxetine (CAS Registry Number: 116539-59-4); milnacipran (CAS Registry Number: 92623-85-3); tramadol (CAS Registry Number: 27203-92-5) and bicifadine (CAS Registry Number: 71195-57-8).

The term "treating" encompasses not only treating a patient to relieve the patient of the signs and symptoms of the disease or condition, or to ameliorate the condition of the patient suffering from the disease or disorder, but also prophylactically treating an asymptomatic patient to prevent the onset or progression of the disease or condition.

The term "patients" include mammalian (including human) patients.

The term "effective amount" refers to an amount of a compound, which confers a therapeutic effect on the treated patient. The effect may be objective (i.e. measurable by some test or marker) or subjective (i.e. the subject gives an indication of or feels an effect).

The invention is illustrated by the following non-limiting Examples:

### EXAMPLES

### Example 1: (R)-1-(5,6-Dichloro-1-(quinolin-2-yl)-1H-benzo[d]imidazol-2-yl)-N-(tetrahydrofuran-3-yl)piperidine-4-carboxamide.

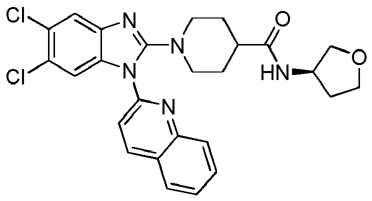

(a) Preparation of the intermediary compound 5,6-dichloro-1*H*-benzo[d]imidazol-2(3*H*)-one: 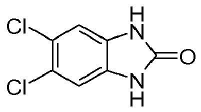 To a stirred solution of 4,5-dichlorobenzene-1,2-diamine (1.0 g, 5.6 mmol) in tetrahydrofuran (10 mL) was added N,N-carbonyl diimidazole (CDI, 1.36g, 8.4 mmol). The mixture was stirred at room temperature for 8 hours under Argon. The reaction mixture was concentrated in vacuo, the residue partitioned between diethyl ether (60 mL) and aqueous sodium hydroxide (60 mL, 1.0 N). The aqueous extract was acidified to reach a pH of approximately 5 by careful addition of aqueous hydrochloric acid (1.5 N). The resulting off-white solid was filtered off, washed with water (3 ×20 mL) and dried under high vacuum to give 0.68 mg (60 % yield) 5,6-dichloro-1*H-*benzo[d]imidazol-2(3H)-one.
(b) Preparation of the intermediary compound 2,5,6-trichloro-1-benzo[d]imidazole: 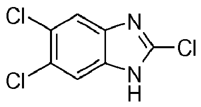 To 5,6-dichloro-1*H*-benzo[d]imidazol-2(3*H*)-one (1.0 g, 4.5 mmol) was added phosphoryl trichloride (4 mL) and the reaction mixture was heated in a screw-cap pressure tube at 120°C for 2 hours. The reaction mixture was concentrated in vacuo, the residue was diluted with water (30 mL) and the resulting off-white solid was filtered off. The precipitate was washed with water (3×20 mL) and dried under high vacuum to afford 0.77 g (71 % yield) of 2,5,6-trichloro-1*H-*benzo[d]imidazole.
(c) Preparation of the intermediate compound ethyl 1-(5,6-dichloro-1H-benzo[d]imidazol-2-yl)piperidine-4-carboxylate: 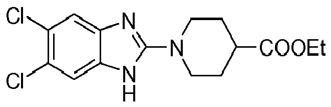 2,5,6-trichloro-1H-benzo[d]imidazole (1 g, 5.0 mmol) was mixed with 1,4-dioxane (9 mL) and ethyl piperidine-4-carboxylate (0.85 g, 5.0 mmol) was added, followed by *N,N-*diisopropylethylamine (Hünig's base, DIEA, 1.034 g, 8 mmol). The reaction mixture was subjected to microwave conditions for one hour at 180°C, purified on column (silica gel, hexane/ethyl acetate, gradient elution, 20-70 % ethyl acetate) to give 1.33 g (87 % yield) of ethyl 1-(5,6-dichloro-1H-benzo[d]imidazol-2-yl)piperidine-4-carboxylate as a brown solid. LC-MS (*m*/*z*) 342.0 (M+1).
(d) Preparation of the intermediate compound 1-(5,6-dichloro-1H-benzo[d]imidazol-2-yl)piperidine-4-carboxylic acid: 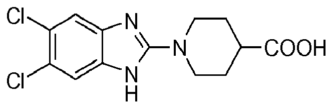 Ethyl 1-(5,6-dichloro-1H-benzo[d]imidazol-2-yl)piperidine-4-carboxylate (0.60 g, 2.0 mmol) was dissolved in ethanol (150 mL) and aqueous sodium hydroxide (1 N, 17.5 mL) was added. The reaction mixture was stirred at room temperature for two days, concentrated in vacuo and pH adjusted to 4-5 with addition of aqueous hydrochloric acid (1 N). The formed precipitate was filtered off, the aqueous mother liquid extracted three times with dichloromethane (100 mL) and the combined organic phases concentrated in vacuo. The crude carboxylic acid (precipitate and the residue from the extraction) was purified on column (silica gel, ethyl acetate/methanol/formic acid, gradient elution 10-15 % methanol, 0.1 % formic acid) to give 0.53 g (90% purity) of 1-(5,6-dichloro-1H-benzo[d]imidazol-2-yl)piperidine-4-carboxylic acid that was used directly in the next step.
(e) Preparation of the intermediate compound (R)-1-(5,6-dichloro-1H-benzo[d]imidazol-2-yl)-N-(tetrahydrofuran-3-yl)piperidine-4-carboxamide: 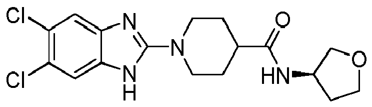 1-(5,6-Dichloro-1H-benzo[d]imidazol-2-yl)piperidine-4-carboxylic acid (628 mg, 2.0 mmol), (R)-(+)-tetrahydrofuran-3-amine 4-methylbenzenesulfonate (622 mg, 2.4 mmol), 2-(7-aza-1H-benzotriazole-1-yl)-1,1,3,3-tetramethyluronium hexafluorophosphate (HATU, 912 mg, 2.4 mmol), N,N-diisopropylethylamine (Hünig's base, DIEA, 775 mg, 6.0 mmol) and N,N-dimethylformamide (25 mL) was stirred at room temperature for 100 minutes. The reaction mixure was concentrated in vacuo, the residue purified on column (silica gel, flash chromatography, dichloromethane/methanol, gradient elution 4-20 % methanol) and finally precipitated from chloroform to give 378 mg (49 % yield) of (R)-1-(5,6-dichloro-1H-benzo[d]imidazol-2-yl)-N-(tetrahydrofuran-3-yl)piperidine-4-carboxamide as a white solid. LC-MS (*m*/*z*) 382.9 (M+1).
(f) A mixture of (R)-1-(5,6-dichloro-1H-benzo[d]imidazol-2-yl)-N-(tetrahydrofuran-3-yl)-piperidine-4-carboxamide (0.14 g, 0.36 mmol), dimethylsulfoxide (2.0 mL), caesium carbonate (0.72 mmol), 2-bromoquinoline (1.1 mmol), 8-hydroxyquinoline (0.14 mmol), polyethylene glycol 400 (0.18mmol) and copper(I) oxide (0.072 mmol) was subjected to microwave conditions for 2.5 hours at 120°C. The reaction mixture was quenched with water (10mL) and extracted with ethyl acetate (2 x30mL). The combined organic layers was washed with water (3 x20 mL), brine (2 x25 mL), dried over anhydrous Na₂SO₄ and concentrated in vacuo. The residue was purified on column (Silica gel, chloroform/methanol gradient elution from 0 to 3 % v/v methanol) to give 35 mg (19% yield) of (*R*)-1-(5,6-dichloro-1-(quinolin-2-yl)-1*H-*benzo[*d*]imidazol-2-yl)-N-(tetrahydrofuran-3-yl)piperidine-4-carboxamide as an off-white solid. LC-MS (*m*/*z*) 510.0 (M+1).

### Example 2: (R)-1-(5-Chloro-6-methyl-1-(quinolin-2-yl)-1H-benzo[d]imidazol-2-yl)-N-(tetrahydrofuran-3-yl)piperidine-4-carboxamide.

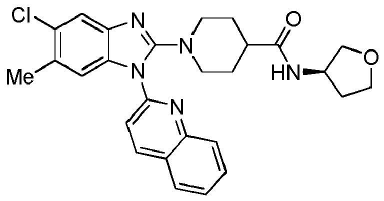

(a) Preparation of the intermediate compound 5-chloro-6-methyl-1*H*-benzo[*d*]imidazol-2(3*H*)-one: 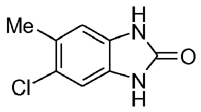 To a stirred solution of 4-chloro-5-methylbenzene-1,2-diamine (0.3 g, 1.9 mmol) in tetrahydrofuran (5 mL) was added *N,N*-carbonyl dimidazole (CDI, 0.465 g, 2.8 mmol). The reaction mixture was stirred at room temperature for 3 hours under Argon gas, concentrated *in vacuo* and the residue partitioned between ethyl acetate (50 mL) and aqueous sodium hydroxide (50 mL, 1 *N*). The aqueous extract was acidified to pH 5 via addition of aqueous hydrochloric acid (1.5 *N*) and the resulting brown precipitate was filtered, washed with water (3 ×20 mL) and dried under high vacuum to obtain 0.28 g (80 % yield) of 5-chloro-6-methyl-1*H*-benzo[*d*]-imidazol-2(3*H*)-one.
(b) Preparation of the intermediary tautomeric compounds 2,5-dichloro-6-methyl-1H-benzo[d]imidazole hydrochloride and 2,6-dichloro-5-methyl-1H-benzo[d]imidazole hydrochloride : 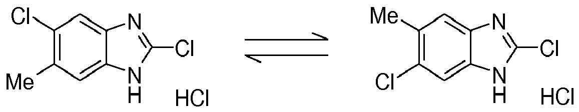 To 5-chloro-6-methyl-1*H*-benzo[*d*]imidazol-2(3*H*)-one (0.627 g, 3.43 mmol), phosphoryl trichloride (POCl₃, 21.1 g, 0.137 mol) was added, the reaction mixture was heated at 100°C for 6 hours and subsequently cooled down to room temperature. The formed precipitate was filtered off and washed with toluene. The filtrate was concentrated *in vacuo* and collected. This gave 674 mg (83 % yield) of 2,5-dichloro-6-methyl-1H-benzo[d]imidazole hydrochloride and 2,6-dichloro-5-methyl-1H-benzo[d]imidazole hydrochloride. LC-MS (*m*/*z*) 200.9 (M+1). No effort was done to to determine the ratio of the tautomers.
(c) Preparation of the intermediary tautomeric compounds ethyl 1-(5-chloro-6-methyl-1H-benzo[d]imidazol-2-yl)piperidine-4-carboxylate and ethyl 1-(6-chloro-5-methyl-1H-benzo[d]imidazol-2-yl)piperidine-4-carboxylate : 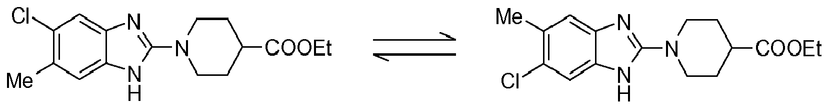 2,5-Dichloro-6-methyl-1H-benzo[d]imidazole hydrochloride and 2,6-dichloro-5-methyl-1H-benzo[d]imidazole hydrochloride (0.60 g, 2.526 mmol), 1,4-dioxane (10 mL), *N,N-*diisopropylethylamine (Hünig's base, DIEA, 0.979 g, 7.578 mmol) and ethyl piperidine-4-carboxylate (0.477 g, 3.03 mmol) was subjected to microwave conditions for one hour at 190°C. The reaction mixture was concentrated in vacuo and purified on column (silica gel, dichloromethane/methanol 98:2) to give 0.498 g (61 % yield) of ethyl 1-(5-chloro-6-methyl-1H-benzo-[d]imidazol-2-yl)piperidine-4-carboxylate and ethyl 1-(6-chloro-5-methyl-1H-benzo[d]imidazol-2-yl)piperidine-4-carboxylate. No effort was done in order to determine the ratios of the tautomers. LC-MS (*m*/*z*) 321.9 (M+1).
(d) Preparation of the intermediate compounds ethyl 1-(5-chloro-6-methyl-1-(quinolin-2-yl)-1H-benzo[d]imidazol-2-yl)piperidine-4-carboxylate and ethyl 1-(6-chloro-5-methyl-1-(quinolin-2-yl)-1H-benzo[d]imidazol-2-yl)piperidine-4-carboxylate: 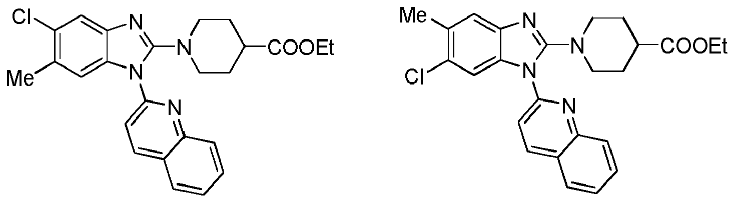 A mixture of the tautomeric compounds ethyl 1-(5-chloro-6-methyl-1*H*-benzo[*d*]imidazol-2-yl)piperidine-4-carboxylate ethyl 1-(6-chloro-5-methyl-1*H*-benzo[*d*]imidazol-2-yl)piperidine-4-carboxylate (100 mg, 0.31 mmol), dimethylsulfoxide (0.7 mL), caesium carbonate (142 mg, 0.44 mmol), 2-chloroquinoline (153 mg, 0.92 mmol), 8-hydroxyquinoline (9.0 mg, 0.062 mmol), polyethylene glycol 400 (62 mg, 0.16 mmol) and copper(I) oxide (8.9 mg, 0.062 mmol) was subjected to microwave conditions for one hour at 170°C. The reaction mixture was diluted with *N,N*-dimethylformamide (5 mL), filtered and concentrated *in vacuo.* The residue was purified by silica flash chromatography (20-50% ethyl acetate in isohexane) to give 30 mg (purity 50%, 11% yield) of the title compound ethyl 1-(5-chloro-6-methyl-1-(quinolin-2-yl)-1*H*-benzo[*d*]imidazol-2-yl)piperidine-4-carboxylate (LC-MS (*m*/*z*) 449.2 (M+1)) eluting at about 30 % ethyl acetate and 10 mg (7 % yield) of the regioisomer ethyl 1-(6-chloro-5-methyl-1-(quinolin-2-yl)-1*H-*benzo[d]imidazol-2-yl)piperidine-4-carboxylate (LC-MS (*m*/*z*) 449.2 (M+1)) eluting at about 35% ethyl acetate.
(e) Preparation of the intermediate compound 1-(5-chloro-6-methyl-1-(quinolin-2-yl)-1*H-*benzo[*d*]imidazol-2-yl)piperidine-4-carboxylic acid: 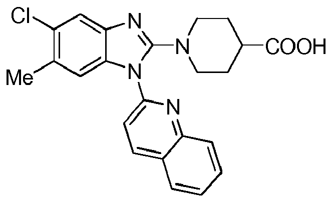 A mixture of ethyl 1-(5-chloro-6-methyl-1-(quinolin-2-yl)-1*H*-benzo[*d*]imidazol-2-yl)piperidine-4-carboxylate (0.033 mmol), ethanol (1.0 mL) water (0.50 mL) and aqueous sodium hydroxide (1 *N*, 0.17 mL, 0.17 mmol) was stirred at 50 °C for 90 minutes. The reaction mixture was neutralized with aqueous hydrochloric acid (2 *N*) and concentrated to dryness in vacuo. All of the salt-containing residue of 1-(5-chloro-6-methyl-1-(quinolin-2-yl)-1*H*-benzo[*d*]imidazol-2-yl)piperidine-4-carboxylic acid was used as is in the next step. LC-MS (*m*/*z*) 421.2 (M+1).
(f) To a mixture of 1-(5-chloro-6-methyl-1-(quinolin-2-yl)-1*H*-benzo[*d*]imidazol-2-yl)piperidine-4-carboxylic acid, (*R*)-(+)-tetrahydro-3-furylamine p-toluenesulfonate (8.6 mg, 0.033 mmol) and N,N-diisopropylethylamine (0.017 mL, 0.10 mmol) in N,N-dimethylformamide (2 mL) was added 2-(7-aza-1H-benzotriazole-1-yl)-1,1,3,3-tetramethyluronium hexafluorophosphate (HATU, 12.6 mg, 0.033 mmol) and the reaction was stirred at room temperature for one hour. The reaction mixture was concentrated in vacuo and the residue purified by silica chromatography (0-10% methanol in ethyl acetate) to give 11.5 mg (71% yield) of (*R*)-1-(5-chloro-6-methyl-1-(quinolin-2-yl)-1*H*-benzo[*d*]imidazol-2-yl)-*N*-(tetrahydrofuran-3-yl)piperidine-4-carboxamide as a white solid. LC-MS (*m*/*z*) 490.3 (M+1).

### Example 3: (R)-1-(6-Chloro-5-methyl-1-(quinolin-2-yl)-1H-benzo[d]imidazol-2-yl)-N (tetrahydrofuran-3-yl)piperidine-4-carboxamide.

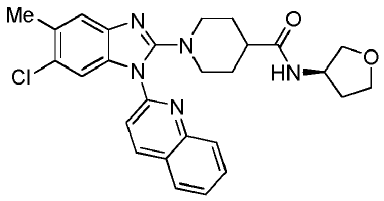

(a) Preparation of the intermediate compound 1-(6-chloro-5-methyl-1-(quinolin-2-yl)-1*H-*benzo[*d*]imidazol-2-yl)piperidine-4-carboxylic acid: 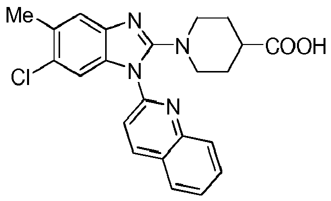 A mixture of ethyl 1-(6-chloro-5-methyl-1-(quinolin-2-yl)-1*H*-benzo[*d*]imidazol-2-yl)piperidine-4-carboxylate (10 mg, 0.023 mmol), ethanol (1.0 mL) water (0.50 mL) and aqueous sodium hydroxide (1 *N*, 0.12 mL, 0.12 mmol) was stirred at 50 °C for 90 minutes. The reaction mixture was neutralized with aqueous hydrochloric acid (2 N) and concentrated to dryness in vacuo. The salt containing residue of 1-(6-chloro-5-methyl-1-(quinolin-2-yl)-1*H*-benzo[*d*]imidazol-2-yl)piperidine-4-carboxylic acid was used as is in the next step. LC-MS (*m*/*z*) 421.2 (M+1).
(b) To a mixture of 1-(6-chloro-5-methyl-1-(quinolin-2-yl)-1*H*-benzo[*d*]imidazol-2-yl)-piperidine-4-carboxylic acid, (*R*)-(+)-tetrahydro-3-furylamine p-toluenesulfonate (6.0 mg, 0.023 mmol, CAS Registry Number: 111769-27-8) and *N,N*-diisopropylethylamine (0.012 mL, 0.069 mmol) in N,N-dimethylformamide (2 mL) was added 2-(7-aza-1H-benzotriazole-1-yl)-1,1,3,3-tetramethyluronium hexafluoro-phosphate (HATU, 9.6 mg, 0.025 mmol) and the reaction was stirred at room temperature for one hour. The reaction mixture was concentrated in vacuo and the residue purified by silica chromatography (0-10% methanol in ethyl acetate) to give 9.4 mg (84% yield) of (*R*)-1-(6-chloro-5-methyl-1-(quinolin-2-yl)-1*H*-benzo[*d*]imidazol-2-yl)-*N-*(tetrahydrofuran-3-yl)piperidine-4-carboxamide as a white solid. LC-MS (*m*/*z*) 490.3 (M+1).

### Example 4: (R)-1-(5,6-Dichloro-1-(6-chloroquinolin-2-yl)-1H-benzo[d]imidazol-2-yl)-N-(tetrahydrofuran-3-yl)piperidine-4-carboxamide.

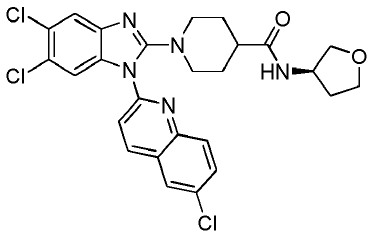

(a) Preparation of the intermediate compound ethyl 1-(5,6-dichloro-1-(6-chloroquinolin-2-yl)-1*H*-benzo[*d*]imidazol-2-yl)piperidine-4-carboxylate: 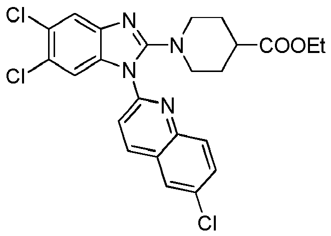 A mixture of ethyl 1-(5,6-dichloro-1*H* benzo[*d*]imidazol-2-yl)piperidine-4-carboxylate (100 mg, 0.29 mmol), dimethylsulfoxide (0.7 mL), caesium carbonate (133 mg, 0.41 mmol), 2,6-dichloroquinoline (174 mg, 0.89 mmol), 8-hydroxyquinoline (8.5 mg, 0.058 mmol), polyethylene glycol 400 (58 mg, 0.15 mmol) and copper(I) oxide (8.4 mg, 0.058 mmol) was subjected to microwave conditions for one hour at 170°C. The reaction mixture was diluted with *N,N*-dimethylformamide (5 mL), filtered and concentrated *in vacuo.* The residue was purified by silica flash chromatography (10-50% ethyl acetate in isohexane) to give 21.1 mg (14% yield) of ethyl 1-(5,6-dichloro-1-(6-chloroquinolin-2-yl)-1*H*-benzo[*d*]imidazo1-2-yl)piperidine-4-carboxylate as a white solid. LC-MS (*m*/*z*) 505.1 (M+1).
(b) Preparation of the intermediate compound 1-(5,6-dichloro-1-(6-chloroquinolin-2-yl)-1*H-*benzo[*d*]imidazol-2-yl)piperidine-4-carboxylic acid: 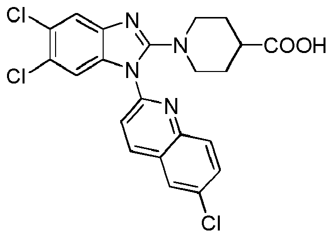 A mixture of ethyl 1-(5,6-dichloro-1-(6-chloroquinolin-2-yl)-1*H*-benzo[*d*]imidazol-2-yl)-piperidine-4-carboxylate (21.1 mg, 0.042 mmol), ethanol (2.0 mL) and aqueous sodium hydroxide (1 N, 0.21 mL, 0.21 mmol) was stirred at 50 °C for 2 hours. The reaction mixture was neutralized with aqueous hydrochloric acid (2 N) and concentrated to dryness in vacuo. The salt-containing residue of 1-(5,6-dichloro-1-(6-chloroquinolin-2-yl)-1*H*-benzo[*d*]imidazol-2-yl)piperidine-4-carboxylic acid was used as is in the next step. LC-MS (*m*/*z*) 477.0 (M+1).
(c) To a mixture of 1-(5,6-dichloro-1-(6-chloroquinolin-2-yl)-1*H*-benzo[*d*]imidazol-2-yl)piperidine-4-carboxylic acid (0.042 mmol), (*R*)-(+)-tetrahydro-3-furylamine p-toluenesulfonate (11 mg, 0.042 mmol) and N,N-diisopropylethylamine (0.021 mL, 0.13 mmol) in N,N-dimethylformamide (2 mL) was added 2-(7-aza-1H-benzotriazole-1-yl)-1,1,3,3-tetramethyluronium hexafluorophosphate (HATU, 17 mg, 0.046 mmol) and the reaction was stirred at room temperature for one hour. The reaction mixture was concentrated in vacuo and the residue purified by silica chromatography (0-8% methanol in ethyl acetate) to give 15.4 mg (68% yield) of (R)-1-(5,6-dichloro-1-(6-chloroquinolin-2-yl)-1*H*-benzo[*d*]imidazol-2-yl)-N (tetrahydrofuran-3-yl)piperidine-4-carboxamide as a white solid. LC-MS (*m*/*z*) 546.1 (M+1).

### Example 5: (R)-1-(5,6-Dichloro-1-(8-chloroquinolin-2-yl)-1H-benzo[d]imidazol-2-yl)-N-(tetrahydrofuran-3-yl)piperidine-4-carboxamide.

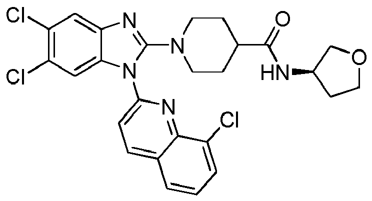

(a) Preparation of the intermediate compound ethyl 1-(5,6-dichloro-1-(8-chloroquinolin-2-yl)-1H-benzo[d]imidazol-2-yl)piperidine-4-carboxylate: 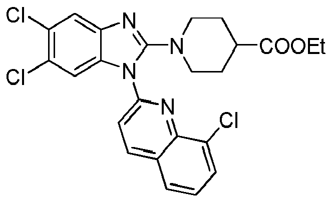 A mixture of ethyl 1-(5,6-dichloro-1*H*-benzo[*d*]imidazol-2-yl)piperidine-4-carboxylate (100 mg, 0.29 mmol), dimethylsulfoxide (0.7 mL), caesium carbonate (133 mg, 0.41 mmol), 2,8-dichloroquinoline (174 mg, 0.89 mmol), 8-hydroxyquinoline (8.5 mg, 0.058 mmol), polyethylene glycol 400 (58 mg, 0.15 mmol) and copper(I) oxide (8.4 mg, 0.058 mmol) was subjected to microwave conditions for one hour at 170°C. The reaction mixture was diluted with *N,N*-dimethylformamide (5 mL), filtered and concentrated in vacuo. The residue was purified by silica flash chromatography (20-50% ethyl acetate in isohexane) to give 49 mg (purity 50%, 17% yield) of ethyl 1-(5,6-dichloro-1-(8-chloroquinolin-2-yl)-1*H*-benzo[*d*]imidazol-2-yl)piperidine-4-carboxylate as a white solid. LC-MS (*m*/*z*) 505.0 (M+1)
(b) Preparation of the intermediate compound 1-(5,6-dichloro-1-(8-chloroquinolin-2-yl)-1*H-*benzo[*d*]imidazol-2-yl)piperidine-4-carboxylic acid: 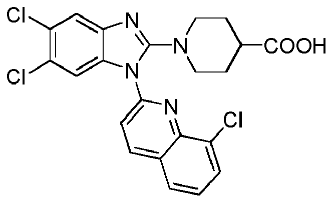 A mixture of ethyl 1-(5,6-dichloro-1-(8-chloroquinolin-2-yl)-1*H* benzo[*d*]imidazol-2-yl)piperidine-4-carboxylate (0.049 mmol), ethanol (2.0 mL) and aqueous sodium hydroxide (1 N, 0.25 mL, 0.25 mmol) was stirred at 50 °C for 2 hours. The reaction mixture was neutralized with aqueous hydrochloric acid (2 N) and concentrated to dryness in vacuo. The salt-containing residue of 1-(5,6-dichloro-1-(8-chloroquinolin-2-yl)-1*H*-benzo[*d*]imidazol-2-yl)piperidine-4-carboxylic acid was used as is in the next step. LC-MS (*m*/*z*) 477.0 (M+1).
(c) To a mixture of 1-(5,6-dichloro-1-(8-chloroquinolin-2-yl)-1*H*-benzo[*d*]imidazol-2-yl)piperidine-4-carboxylic acid (0.049 mmol), (*R*)-(+)-tetrahydro-3-furylamine p-toluenesulfonate (13 mg, 0.049 mmol) and *N,N*-diisopropylethylamine (0.025 mL, 0.15 mmol) in N,N-dimethylformamide (2 mL) was added 2-(7-aza-1H-benzotriazole-1-yl)-1,1,3,3-tetramethyluronium hexafluorophosphate (HATU, 20 mg, 0.054 mmol) and the reaction was stirred at room temperature for 1 hour. The reaction mixture was concentrated in vacuo and the residue purified by silica chromatography (0-10% methanol in ethyl acetate) to give 18.7 mg (70% yield) of (*R*)-1-(5,6-dichloro-1-(8-chloroquinolin-2-yl)-1*H* benzo[d]imidazol-2-yl)-*N-*(tetrahydrofuran-3-yl)piperidine-4-carboxamide as a white solid. LC-MS (*m*/*z*) 546.1 (M+1).

### Example 6: (R)-1-(1-(6-Chloroquinolin-2-yl)-5,6-dimethyl-1H-benzo[d]imidazol-2-yl)-N-(tetrahydrofuran-3-yl)piperidine-4-carboxamide.

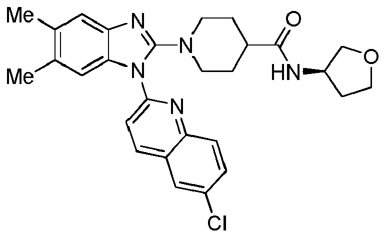

(a) Preparation of the intermediary compound 2-chloro-5,6-dimethyl-1*H*-benzo[*d*]imidazole: 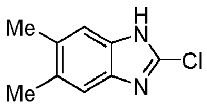 To a solution of sodium nitrite (3.77 g, 54.6 mmol) in water (220 mL) was added an aqueous solution of copper(II) chloride (12.24 g, 91.06 mmol, in 160 mL of water) The mixture was stirred at room temperature for 5 minutes and 5,6-dimethyl-1*H*-benzo[d]imidazol-2-amine (2.936 g, 18.21 mmol) was added to the mixture in portions for 5 minutes. The reaction mixture was stirred at room temperature for one hour, heated at 100 °C for one hour and cooled down. The resulting reaction mixture was extracted with ethyl acetate (3 x300 mL), the combined organic phases washed with brine (200 mL) and dried over anhydrous magnesium sulphate. After concentration *in vacuo,* the residue was subjected to column chromathography (silica gel, methanol/dichloromethane gradient elution consisting of 1-5% methanol), to give 1.054 g (29 % yield: calculated on 90 % purity according to LC-MS) of 2-chloro-5,6-dimethyl-1*H-*benzo[d]imidazole that was used directly in the next step.
(b) Preparation of the intermediate compound ethyl 1-(5,6-dimethyl-1*H*-benzo[d]imidazol-2-yl)piperidine-4-carboxylate: 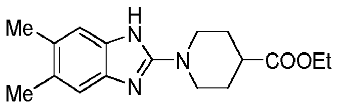 2-Chloro-5,6-dimethyl-1H-benzo[d]imidazole (180.6 mg, 1 mmol), ethyl piperidine-4-carboxylate (157.2, 1 mmol) and 1,4-dioxane (4 mL) were mixed in a 5 mL microwave vial and irradiated under microwave conditions at 180°C for 1 hour. The reaction mixture was concentrated in vacuo, subjected to column chromatography (silica gel, dichloromethane/methanol 90:10), to give 288 mg (96 % yield) of ethyl 1-(5,6-dimethyl-1H-benzo[d]imidazol-2-yl)piperidine-4-carboxylate. LC-MS (*m*/*z*) 302.2 (M+1).
(c) Preparation of the intermediate compound ethyl 1-(1-(6-chloroquinolin-2-yl)-5,6-dimethyl-1*H*-benzo[*d*]imidazol-2-yl)piperidine-4-carboxylate: 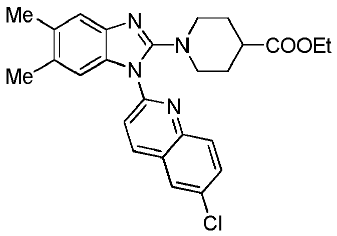 A mixture of ethyl 1-(5,6-dimethyl-1*H*-benzo[*d*]imidazol-2-yl)piperidine-4-carboxylate (90 mg, 0.30 mmol), dimethylsulfoxide (0.7 mL), caesium carbonate (136 mg, 0.42 mmol), 2,6-dichloroquinoline (177 mg, 0.90 mmol), 8-hydroxyquinoline (8.7 mg, 0.060 mmol), polyethylene glycol 400 (60 mg, 0.15 mmol) and copper(I) oxide (8.5 mg, 0.060 mmol) was subjected to microwave conditions for one hour at 170 °C. The reaction mixture was diluted with *N,N*-dimethylformamide (5 mL), filtered and concentrated in vacuo. The residue was purified by silica flash chromatography (20-50% ethyl acetate in isohexane) to give 14.1 mg (10% yield) of ethyl 1-(1-(6-chloroquinolin-2-yl)-5,6-dimethyl-1*H*-benzo[*d*]imidazol-2-yl)piperidine-4-carboxylate as a white solid. LC-MS (*m*/*z*) 463.2 (M+1).
(d) Preparation of the intermediate compound 1-(1-(6-chloroquinolin-2-yl)-5,6-dimethyl-1*H-*benzo[*d*]imidazol-2-yl)piperidine-4-carboxylic acid: 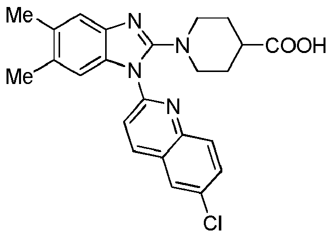 A mixture of ethyl 1-(1-(6-chloroquinolin-2-yl)-5,6-dimethyl-1*H*-benzo[d]imidazol-2-yl)piperidine-4-carboxylate (14.1 mg, 0.031 mmol), ethanol (2.0 mL) and aqueous sodium hydroxide (1 N, 0.15 mL, 0.15 mmol) was stirred at 50 °C for 2 hours. The reaction mixture was neutralized with aqueous hydrochloric acid (2 N) and concentrated to dryness *in vacuo.* The whole salt-containing residue of 1-(1-(6-chloroquinolin-2-yl)-5,6-dimethyl-1*H*-benzo[*d*]-imidazol-2-yl)piperidine-4-carboxylic acid was used as is in the next step. LC-MS (*m*/*z*) 435.2 (M+1).
(e) To a mixture of 1-(1-(6-chloroquinolin-2-yl)-5,6-dimethyl-1*H*-benzo[*d*]imidazol-2-yl)piperidine-4-carboxylic acid, (*R*)-(+)-tetrahydro-3-furylamine p-toluenesulfonate (7.9 mg, 0.031mmol) and *N,N*-diisopropylethylamine (0.016 mL, 0.091 mmol) in N,N-dimethylformamide (2 mL) was added 2-(7-aza-1H-benzotriazole-1-yl)-1,1,3,3-tetramethyluronium hexafluorophosphate (HATU, 13 mg, 0.034 mmol) and the reaction was stirred at room temperature for 80 minutes. The reaction mixture was concentrated in vacuo and the residue purified by silica chromatography (0-10% methanol in ethyl acetate) to give 7.8 mg (51% yield) of (*R*)-1-(1-(6-chloroquinolin-2-yl)-5,6-dimethyl-1*H*-benzo[d]imidazol-2-yl)-*N-*(tetrahydrofuran-3-yl)piperidine-4-carboxamide as a white solid. LC-MS (*m*/*z*) 504.2 (M+1).

### Example 7: (R)-1-(5,6-Dichloro-1-(4-methylquinolin-2-yl)-1H-benzo[d]imidazol-2-yl)-N-(tetrahydrofuran-3-yl)piperidine-4-carboxamide.

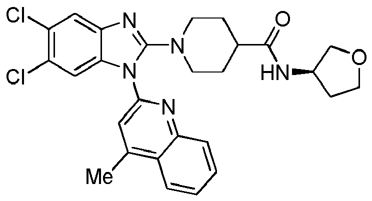

(a) Preparation of the intermediate compound 1-(5,6-dichloro-1-(4-methylquinolin-2-yl)-1H-benzo[d]imidazol-2-yl)piperidine-4-carboxylic acid: 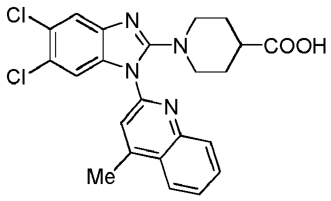 A mixture of ethyl 1-(5,6-dichloro-1*H*-benzo[*d*]imidazol-2-yl)piperidine-4-carboxylate (100 mg, 0.29 mmol), dimethylsulfoxide (0.7 mL), caesium carbonate (133 mg, 0.41 mmol), 2-chloro-4-methylquinoline (156 mg, 0.88 mmol), 8-hydroxyquinoline (8.5 mg, 0.058 mmol), polyethylene glycol 400 (58 mg, 0.15 mmol) and copper(I) oxide (8.4 mg, 0.058 mmol) was subjected to microwave conditions for one hour at 170 °C. LC-MS indicated partial hydrolysis of the expected ester product. The reaction mixture was diluted with *N,N*-dimethylformamide (5 mL), filtered and concentrated *in vacuo.* The residue was purified by silica flash chromatography (20-70 % ethyl acetate in isohexane) to give 32 mg (purity 50%, 11% yield) of ethyl 1-(5,6-dichloro-1-(4-methylquinolin-2-yl)-1*H*-benzo[*d*]imidazol-2-yl)piperidine-4-carboxylate as a white solid. LC-MS (*m*/*z*) 483.1 (M+1). The silica column was subsequently eluted with 0.5% formic acid in ethyl acetate to give 20 mg (12% yield calculated on 80 % purity according to LC-MS) of 1-(5,6-dichloro-1-(4-methylquinolin-2-yl)-1H-benzo[d]imidazol-2-yl)piperidine-4-carboxylic acid as a white solid. LC-MS (*m*/*z*) 455.1 (M+1).
(b) To a mixture of 1-(5,6-dichloro-1-(4-methylquinolin-2-yl)-1H-benzo[d]imidazol-2-yl)-piperidine-4-carboxylic acid (0.035 mmol, amount calculated on 80 % purity according to LC-MS), (*R*)-(+)-tetrahydro-3-furylamine p-toluenesulfonate (9.0 mg, 0.035 mmol)and *N,N-*diisopropylethylamine (0.012 mL, 0.070 mmol) in N,N-dimethylformamide (2 mL) was added 2-(7-aza-1H-benzotriazole-1-yl)-1,1,3,3-tetramethyluronium hexafluorophosphate (HATU, 14.5 mg, 0.038 mmol) and the reaction was stirred at room temperature for one hour. The reaction mixture was concentrated in vacuo and the residue purified by silica chromatography (0-10% methanol in ethyl acetate) to give 14.3 mg (78% yield) of (*R*)-1-(5,6-dichloro-1-(4-methylquinolin-2-yl)-1*H*-benzo[*d*]imidazol-2-yl)-*N*-(tetrahydrofuran-3-yl)piperidine-4-carboxamide as a white solid. LC-MS (*m*/*z*) 524.2 (M+1).

### Example 8: (R)-1-(5-Chloro-1-(6-chloroquinolin-2-yl)-6-methyl-1H-benzo[d]imidazol-2-yl)-N-(tetrahydrofuran-3-yl)piperidine-4-carboxamide.

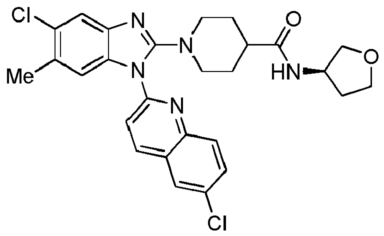

(a) Preparation of the intermediate compounds ethyl 1-(5-chloro-1-(6-chloroquinolin-2-yl)-6-methyl-1H-benzo[d]imidazol-2-yl)piperidine-4-carboxylate: 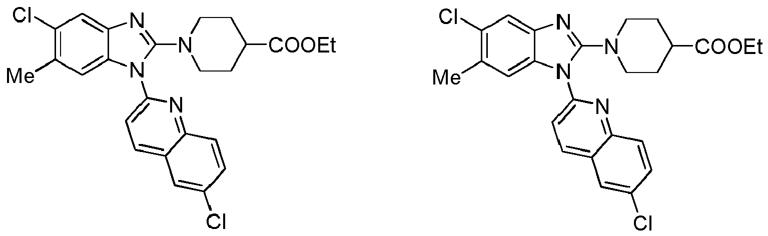 A mixture of the tautomeric compounds ethyl 1-(5-chloro-6-methyl-1*H*-benzo[d]imidazol-2-yl)piperidine-4-carboxylate ethyl 1-(6-chloro-5-methyl-1*H*-benzo[*d*]imidazol-2-yl)piperidine-4-carboxylate (100 mg, 0.31 mmol), dimethylsulfoxide (0.7 mL), caesium carbonate (142 mg, 0.44 mmol), 2,6-dichloroquinoline (185 mg, 0.93 mmol), 8-hydroxyquinoline (9.0 mg, 0.062 mmol), polyethylene glycol 400 (62 mg, 0.16 mmol) and copper(I) oxide (8.9 mg, 0.062 mmol) was subjected to microwave conditions for one hour at 170 °C. The reaction mixture was diluted with *N,N*-dimethylformamide (5 mL), filtered and concentrated *in vacuo.* The residue was purified by silica flash chromatography (10-50% ethyl acetate in isohexane) to give 18 mg (purity 50%, 6% yield) of ethyl 1-(5-chloro-1-(6-chloroquinolin-2-yl)-6-methyl-1H-benzo[d]imidazol-2-yl)piperidine-4-carboxylate (LC-MS (*m*/*z*) 483.1 (M+1)) eluting at about 32% ethyl acetate and 8.4 mg (6% yield) of the regioisomer ethyl 1-(6-chloro-1-(6-chloroquinolin-2-yl)-5-methyl-1H-benzo[d]imidazol-2-yl)piperidine-4-carboxylate (LC-MS (*m*/*z*) 483.1 (M+1)), eluting at about 38% ethyl acetate.
(b) Preparation of the intermediate compound 1-(5-chloro-1-(6-chloroquinolin-2-yl)-6-methyl-1H-benzo[d]imidazol-2-yl)piperidine-4-carboxylic acid: 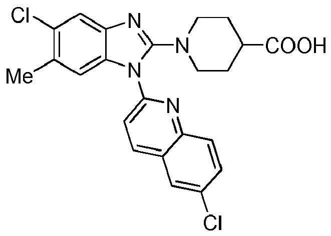 A mixture of ethyl 1-(5-chloro-1-(6-chloroquinolin-2-yl)-6-methyl-1H-benzo[d]imidazol-2-yl)piperidine-4-carboxylate (0.019 mmol), ethanol (2.0 mL) and aqueous sodium hydroxide (1 N, 0.093 mL, 0.093 mmol) was stirred at 50 °C for 90 minutes. The reaction mixture was neutralized with aqueous hydrochloric acid (2 N) and concentrated to dryness in vacuo. All of the the salt-containing residue of 1-(5-chloro-1-(6-chloroquinolin-2-yl)-6-methyl-1H-benzo[d]imidazol-2-yl)piperidine-4-carboxylic acid was used as is in the next step. LC-MS (*m*/*z*) 455.1 (M+1).
(c) To a mixture of 1-(5-chloro-1-(6-chloroquinolin-2-yl)-6-methyl-1H-benzo[d]imidazol-2-yl)piperidine-4-carboxylic acid, (*R*)-(+)-tetrahydro-3-furylamine p-toluenesulfonate (4.8 mg, 0.019 mmol)and N,N-diisopropylethylamine (9.6 µL, 0.056 mmol) in N,N-dimethylformamide (2 mL) was added 2-(7-aza-1H-benzotriazole-1-yl)-1,1,3,3-tetramethyluronium hexafluorophosphate (HATU, 7.8 mg, 0.021 mmol) and the reaction was stirred at room temperature for 90 minutes. The reaction mixture was concentrated in vacuo and the residue purified by silica chromatography (0-10% methanol in ethyl acetate) to give 6.4 mg (65% yield) of (R)-1-(5-chloro-1-(6-chloroquinolin-2-yl)-6-methyl-1*H*-benzo[*d*]imidazol-2-yl)-*N-*(tetrahydrofuran-3-yl)piperidine-4-carboxamide as a white solid. LC-MS (*m*/*z*) 524.1 (M+1).

### Example 9: (R)-1-(6-Chloro-1-(6-chloroquinolin-2-yl)-5-methyl-1H-benzo[d]imidazol-2-yl)-N-(tetrahydrofuran-3-yl)piperidine-4-carboxamide.

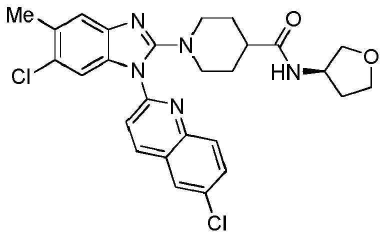

(a) Preparation of the intermediate compound 1-(6-chloro-1-(6-chloroquinolin-2-yl)-5-methyl-1*H*-benzo[*d*]imidazol-2-yl)piperidine-4-carboxylic acid: 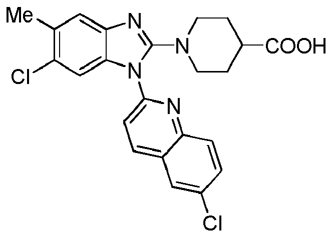 A mixture of ethyl 1-(6-chloro-1-(6-chloroquinolin-2-yl)-5-methyl-1*H*-benzo[*d*]imidazol-2-yl)piperidine-4-carboxylate (0.017mmol), ethanol (2.0 mL) and aqueous sodium hydroxide (1 N, 0.087 mL, 0.087 mmol) was stirred at 50 °C for 90 minutes. The reaction mixture was neutralized with aqueous hydrochloric acid (2 N) and concentrated to dryness in vacuo. The salt-containing residue of 1-(6-chloro-1-(6-chloroquinolin-2-yl)-5-methyl-1*H*-benzo[*d*]imidazol-2-yl)piperidine-4-carboxylic acid was used as is in the next step. LC-MS (*m*/*z*) 455.1 (M+1).
(b) To a mixture of 1-(6-chloro-1-(6-chloroquinolin-2-yl)-5-methyl-1*H*-benzo[*d*]imidazol-2-yl)piperidine-4-carboxylic acid, (*R*)-(+)-tetrahydro-3-furylamine p-toluenesulfonate (4.5 mg, 0.017 mmol) and N,N-diisopropylethylamine (9.0 µL, 0.052 mmol) in N,N-dimethylformamide (2 mL) was added 2-(7-aza-1H-benzotriazole-1-yl)-1,1,3,3-tetramethyluronium hexafluorophosphate (HATU, 7.3 mg, 0.019 mmol) and the reaction was stirred at room temperature for 90 minutes. The reaction mixture was concentrated in vacuo and the residue purified by silica chromatography (0-10% methanol in ethyl acetate) to give 6.4 mg (70% yield) of (R)-1-(6-chloro-1-(6-chloroquinolin-2-yl)-5-methyl-1*H*-benzo[*d*]imidazol-2-yl)-N-(tetrahydrofuran-3-yl)piperidine-4-carboxamide as a white solid. LC-MS (*m*/*z*) 524.1 (M+1).

### Example 10: (R)-1-(5,6-Dichloro-1-(6-fluoroquinolin-2-yl)-1H-benzo[d]imidazol-2-yl)-N-(tetrahydrofuran-3-yl)piperidine-4-carboxamide.

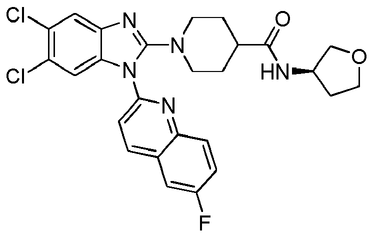

(a) Preparation of the intermediate compound 1-(5,6-dichloro-1-(6-fluoroquinolin-2-yl)-1*H-*benzo[*d*]imidazol-2-yl)piperidine-4-carboxylic acid, and ethyl 1-(5,6-dichloro-1-(6-fluoroquinolin-2-yl)-1H-benzo [d]imidazol-2-yl)piperidine-4-carboxylate: 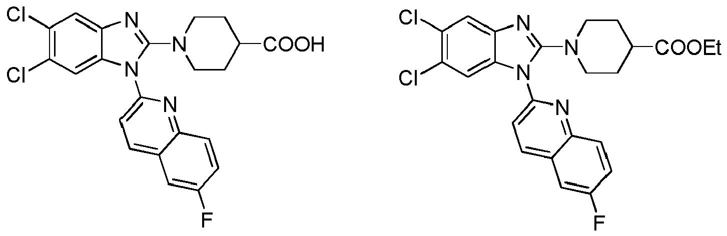 A mixture of ethyl 1-(5,6-dichloro-1*H*-benzo[*d*]imidazol-2-yl)piperidine-4-carboxylate (100 mg, 0.29 mmol), dimethylsulfoxide (0.7 mL), caesium carbonate (133 mg, 0.41 mmol), 2-chloro-6-fluoroquinoline (159 mg, 0.88 mmol), 8-hydroxyquinoline (8.5 mg, 0.058 mmol), polyethylene glycol 400 (58 mg, 0.15 mmol) and copper(I) oxide (8.4 mg, 0.058 mmol) was subjected to microwave conditions for one hour at 170°C. LCMS indicated partial hydrolysis of the expected ester product. The reaction mixture was diluted with *N,N*-dimethylformamide (5 mL), filtered and concentrated in vacuo. The residue was purified by silica flash chromatography (10-50% ethyl acetate in isohexane) to give 34 mg (15% yield calculated on 65 % purity according to LC-MS) of ethyl 1-(5,6-dichloro-1-(6-fluoroquinolin-2-yl)-1*H*-benzo[*d*]imidazol-2-yl)piperidine-4-carboxylate as a white solid. LC-MS (*m*/*z*) 487.1 (M+1). The silica column was then eluted with 0-1% formic acid in ethyl acetate to give 14 mg (9% yield calculated on 80 % purity according to LC-MS) of 1-(5,6-dichloro-1-(6-fluoroquinolin-2-yl)-1H-benzo[d]-imidazol-2-yl)piperidine-4-carboxylic acid as a white solid. LC-MS (*m*/*z*) 459.0 (M+1).
(b) To a mixture of 1-(5,6-dichloro-1-(6-fluoroquinolin-2-yl)-1H-benzo[d]imidazol-2-yl)piperidine-4-carboxylic acid (0.025 mmol), (*R*)-(+)-tetrahydro-3-furylamine p-toluenesulfonate (6.5 mg, 0.025 mmol)and *N,N*-diisopropylethylamine (0.013 mL, 0.075 mmol) in N,N-dimethylformamide (2 mL) was added 2-(7-aza-1H-benzotriazole-1-yl)-1,1,3,3-tetramethyluronium hexafluorophosphate (HATU, 10.4 mg, 0.027 mmol) and the reaction was stirred at room temperature for 1 hour. The reaction mixture was concentrated in vacuo and the residue purified by silica chromatography (0-10% methanol in ethyl acetate) to give 8.3 mg (63% yield) of (*R*)-1-(5,6-dichloro-1-(6-fluoroquinolin-2-yl)-1*H*-benzo[*d*]imidazol-2-yl)-*N-*(tetrahydrofuran-3-yl)piperidine-4-carboxamide as a white solid. LC-MS (*m*/*z*) 528.1 (M+1).

### Example 11: (R)-1-(5-Chloro-1-(6-fluoroquinolin-2-yl)-6-methyl-1H-benzo[d]imidazol-2-yl)-N-(tetrahydrofuran-3-yl)piperidine-4-carboxamide.

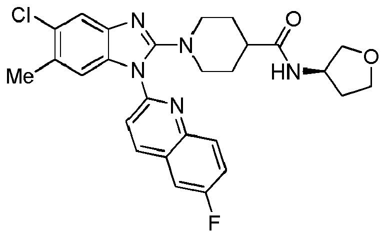

(a) Preparation of the intermediate compounds ethyl 1-(5-chloro-1-(6-fluoroquinolin-2-yl)-6-methyl-1H-benzo[d]imidazol-2-yl)piperidine-4-carboxylate and ethyl 1-(6-chloro-1-(6-fluoroquinolin-2-yl)-5-methyl-1H-benzo[d]imidazol-2-yl)piperidine-4-carboxylate: 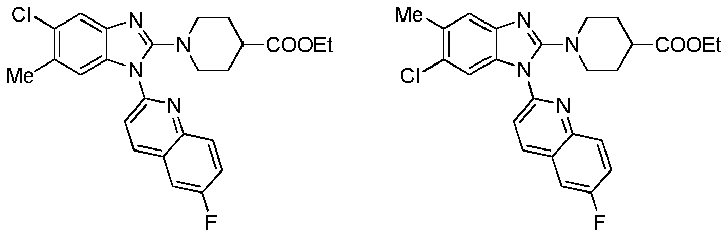 A mixture of ethyl 1-(5-chloro-6-methyl-1*H*-benzo[*d*]imidazol-2-yl)piperidine-4-carboxylate (100 mg, 0.31 mmol), dimethylsulfoxide (0.7 mL), caesium carbonate (142 mg, 0.44 mmol), 2-chloro-6-fluoroquinoline (169mg, 0.93 mmol), 8-hydroxyquinoline (9.0 mg, 0.062 mmol), polyethylene glycol 400 (62 mg, 0.16 mmol) and copper(I) oxide (8.9 mg, 0.062 mmol) was subjected to microwave conditions for one hour at 170 °C. The reaction mixture was diluted with *N,N*-dimethylformamide (5 mL), filtered and concentrated in vacuo. The residue was purified by silica flash chromatography (25-45% ethyl acetate in isohexane) to give 37 mg (13 % yield calculated on 50 % purity, according to LC-MS) of ethyl 1-(5-chloro-1-(6-fluoroquinolin-2-yl)-6-methyl-1H-benzo[d]imidazol-2-yl)piperidine-4-carboxylate (LC-MS (*m*/*z*) 467.2 (M+1)) eluting at about 30% ethyl acetate and 14 mg (10% yield) of the regioisomer ethyl 1-(6-chloro-l-(6-fluoroquinolin-2-yl)-5-methyl-1*H*-benzo[*d*]imidazol-2-yl)piperidine-4-carboxylate (LC-MS (*m*/*z*) 467.2 (M+1)) eluting at about 38% ethyl acetate.
(b) Preparation of the intermediate compound 1-(5-chloro-1-(6-fluoroquinolin-2-yl)-6-methyl-1*H*-benzo[*d*]imidazol-2-yl)piperidine-4-carboxylic acid: 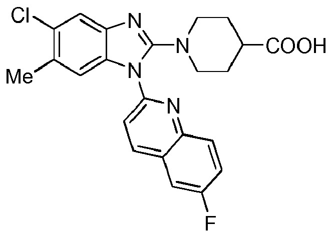 A mixture of ethyl 1-(5-chloro-1-(6-fluoroquinolin-2-yl)-6-methyl-1H-benzo[d]imidazol-2-yl)piperidine-4-carboxylate (0.040 mmol), ethanol (2.0 mL) and aqueous sodium hydroxide (1 N, 0.20 mL, 0.20 mmol) was stirred at 50 °C for 60 minutes. The reaction mixture was neutralized with aqueous hydrochloric acid (2 N) and concentrated to dryness in vacuo. All of the salt-containing residue of 1-(5-chloro-1-(6-fluoroquinolin-2-yl)-6-methyl-1H-benzo[d]imidazol-2-yl)piperidine-4-carboxylic acid was used as is in the next step. LC-MS (*m*/*z*) 439.1 (M+1).
(c) To a mixture of 1-(5-chloro-1-(6-fluoroquinolin-2-yl)-6-methyl-1H-benzo[d]imidazol-2-yl)piperidine-4-carboxylic acid, (*R*)-(+)-tetrahydro-3-furylamine p-toluenesulfonate (10 mg, 0.040 mmol)and N,N-diisopropylethylamine (20 µL, 0.12 mmol) in N,N-dimethylformamide (2 mL) was added 2-(7-aza-1H-benzotriazole-1-yl)-1,1,3,3-tetramethyluronium hexafluorophosphate (HATU, 17mg, 0.044 mmol) and the reaction was stirred at room temperature for 60 minutes. The reaction mixture was concentrated in vacuo and the residue purified by silica chromatography (2-10% methanol in ethyl acetate) to give 11 mg (54% yield) of (*R*)-1-(5-chloro-1-(6-fluoroquinolin-2-yl)-6-methyl-1*H*-benzo[*d*]imidazol-2-yl)-*N-*(tetrahydrofuran-3-yl)piperidine-4-carboxamide as a white solid. LC-MS (*m*/*z*) 508.2 (M+1).

### Example 12: (R)-1-(6-Chloro-1-(6-fluoroquinolin-2-yl)-5-methyl-1H-benzo[d]imidazol-2-yl)-N-(tetrahydrofuran-3-yl)piperidine-4-carboxamide.

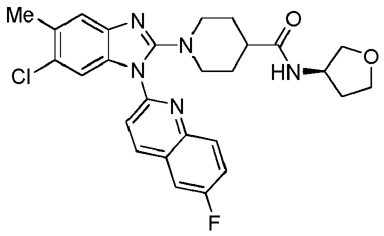

(a) Preparation of the intermediate compound 1-(6-chloro-1-(6-fluoroquinolin-2-yl)-5-methyl-1*H*-benzo[*d*]imidazol-2-yl)piperidine-4-carboxylic acid: 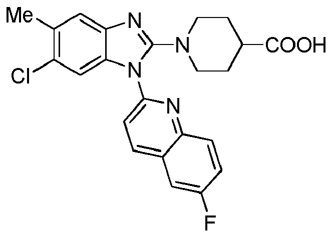 A mixture of ethyl 1-(6-chloro-1-(6-fluoroquinolin-2-yl)-5-methyl-1*H*-benzo[*d*]imidazol-2-yl)piperidine-4-carboxylate (0.030 mmol), ethanol (2.0 mL) and aqueous sodium hydroxide (1 N, 0.15 mL, 0.15 mmol) was stirred at 50 °C for 60 minutes. The reaction mixture was neutralized with aqueous hydrochloric acid (2 N) and concentrated to dryness in vacuo. All of the salt-containing residue of 1-(6-chloro-1-(6-fluoroquinolin-2-yl)-5-methyl-1*H-*benzo[*d*]imidazol-2-yl)piperidine-4-carboxylic acid was used as is in the next step. LC-MS (*m*/*z*) 439.1 (M+1).
(b) To a mixture of 1-(6-chloro-1-(6-fluoroquinolin-2-yl)-5-methyl-1*H*-benzo[*d*]imidazol-2-yl)piperidine-4-carboxylic acid, (*R*)-(+)-tetrahydro-3-furylamine p-toluenesulfonate (7.8 mg, 0.030 mmol)and N,N-diisopropylethylamine (16 µL, 0.091 mmol) in N,N-dimethylformamide (2 mL) was added 2-(7-aza-1H-benzotriazole-1-yl)-1,1,3,3-tetramethyluronium hexafluorophosphate (HATU, 13mg, 0.033 mmol) and the reaction was stirred at room temperature for 60 minutes. The reaction mixture was concentrated in vacuo and the residue purified by silica chromatography (2-10% methanol in ethyl acetate) to give 11.5 mg (75% yield) of (R)-1-(6-chloro-1-(6-fluoroquinolin-2-yl)-5-methyl-1*H*-benzo[*d*]imidazol-2-yl)-*N*-(tetrahydrofuran-3-yl)piperidine-4-carboxamide as a white solid. LC-MS (*m*/*z*) 508.2 (M+1).

### Example 13: (R)-1-(5,6-Dichloro-1-(8-fluoroquinolin-2-yl)-1H-benzo[d]imidazol-2-yl)-N-(tetrahydrofuran-3-yl)piperidine-4-carboxamide.

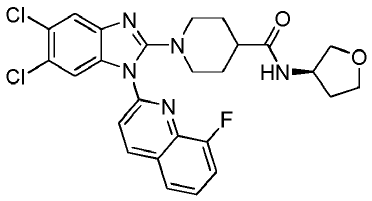

(a) Preparation of the intermediate compound 1-(5,6-dichloro-1-(8-fluoroquinolin-2-yl)-1*H-*benzo[*d*]imidazol-2-yl)piperidine-4-carboxylic acid, and ethyl 1-(5,6-dichloro-1-(8-fluoroquinolin-2-yl)-1H-benzo [d]imidazol-2-yl)piperidine-4-carboxylate: 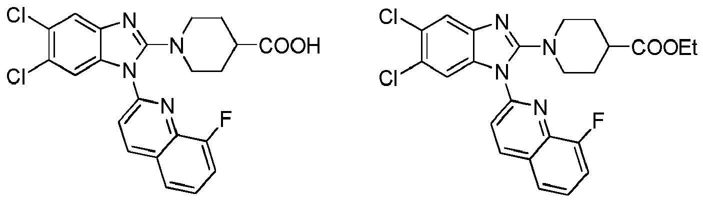 A mixture of ethyl 1-(5,6-dichloro-1*H*-benzo[*d*]imidazol-2-yl)piperidine-4-carboxylate (100 mg, 0.29 mmol), dimethylsulfoxide (0.7 mL), caesium carbonate (133 mg, 0.41 mmol), 2-chloro-8-fluoroquinoline (159 mg, 0.88 mmol), 8-hydroxyquinoline (8.5 mg, 0.058 mmol), polyethylene glycol 400 (58 mg, 0.15 mmol) and copper(I) oxide (8.4 mg, 0.058 mmol) was subjected to microwave conditions for one hour at 170°C. LCMS indicated partial hydrolysis of the expected ester product. The reaction mixture was diluted with *N,N*-dimethylformamide (5 mL), filtered and concentrated in vacuo. The residue was purified by silica flash chromatography (25-50% ethyl acetate in isohexane) to give 24 mg (13% yield calculated on 75 % purity, according to LC-MS) of ethyl 1-(5,6-dichloro-1-(8-fluoroquinolin-2-yl)-1*H*-benzo[*d*]imidazol-2-yl)piperidine-4-carboxylate as a white solid. LC-MS (*m*/*z*) 487.1 (M+1). The silica column was then eluted with 0-1% formic acid in ethyl acetate to give 9.4 mg (4% yield calculated on 60 % purity, according to LC-MS) of 1-(5,6-dichloro-1-(8-fluoroquinolin-2-yl)-1H-benzo[d]imidazol-2-yl)piperidine-4-carboxylic acid as a yellow sold. LC-MS (*m*/*z*) 459.0 (M+1).
(b) To a mixture of 1-(5,6-dichloro-1-(8-fluoroquinolin-2-yl)-1H-benzo[d]imidazol-2-yl)-piperidine-4-carboxylic acid (0.012 mmol), (*R*)-(+)-tetrahydro-3-furylamine p-toluenesulfonate (3.2 mg, 0.012 mmol) and *N,N*-diisopropylethylamine (6 µL, 0.037 mmol) in N,N-dimethylformamide (1.0 mL) was added 2-(7-aza-1H-benzotriazole-1-yl)-1,1,3,3-tetramethyluronium hexafluorophosphate (HATU, 5.1 mg, 0.014 mmol) and the reaction was stirred at room temperature for one hour. The reaction mixture was concentrated in vacuo and the residue purified by silica chromatography (0-10 % methanol in ethyl acetate) to give 2.8 mg (43% yield) of (*R*)-1-(5,6-dichloro-1-(8-fluoroquinolin-2-yl)-1*H*-benzo[*d*]imidazol-2-yl)-*N*-(tetrahydrofuran-3-yl)piperidine-4-carboxamide as a light yellow solid. LC-MS (*m*/*z*) 528.1 (M+1).

### Example 14: (R)-1-(5,6-Dichloro-1-(isoquinolin-3-yl)-1H-benzo[d]imidazol-2-yl)-N-(tetrahydrofuran-3-yl)piperidine-4-carboxamide.

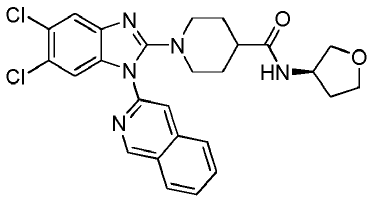

A mixture of (R)-1-(5,6-dichloro-1H-benzo[d]imidazol-2-yl)-N-(tetrahydrofuran-3-yl)-piperidine-4-carboxamide (0.1 g, 0.261 mmol), dimethylsulfoxide (4.0 mL), caesium carbonate (0.104 mmol), 2-bromoquinoline (0.78 mmol), 8-hydroxyquinoline (0.14 mmol), polyethylene glycol 400 (0.3 mL) and copper(I) oxide (0.0522 mmol) was subjected to microwave conditions for three hours at 120°C. The reaction mixture was quenched with water (10 mL) and extracted with ethyl acetate (2 x30 mL). The combined organic layers was washed with water (2 x20 mL), brine (2 x25 mL), dried over anhydrous sodium carbonate and concentrated in vacuo. The residue was purified on column (Silica gel, chloroform/methanol 98:2) to give 10 mg (8 % yield) of (R)-1-(5,6-dichloro-1-(isoquinolin-3-yl)-1H-benzo[d]imidazol-2-yl)-N-(tetrahydrofuran-3-yl)-piperidine-4-carboxamide as an pale brown solid. LC-MS (*m*/*z*) 510.3 (M+1).

Below follows two comparative examples that were synthesized and tested in parallel with the examples of the invention.

### Comparative Example 15: (R)-1-(5,6-dimethyl-1-(quinolin-6-yl)-1H-benzo[d]imidazol-2-yl)-N-(tetrahydrofuran-3-yl)piperidine-4-carboxamide:

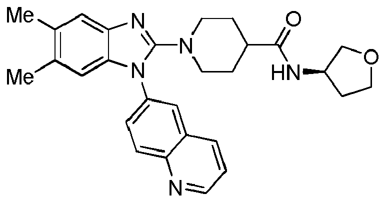

(a) Preparation of the intermediary compound ethyl 1-(5,6-dimethyl-1*H*-benzo[*d*]imidazol-2-yl)piperidine-4-carboxylate: 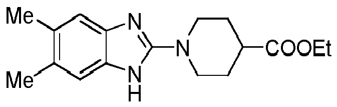 2-Chloro-5,6-dimethyl-1*H*-benzo[*d*]imidazole (1 g, 5.53 mmol) was mixed with 1,4-dioxane (10 mL) and ethyl piperidine-4-carboxylate (8.30 mmol) was added, followed by *N,N*-diisopropylethylamine (Hünig's base, DIEA, 16.59 mmol). The reaction mixture was subjected to microwave conditions for three hours at 180 °C, poured into ice water, stirred for 10 minutes, the resultant precipitate filtered off and *dried in vacuo.* The solid was recrystallized from petroleum ether (40 mL) to give 1.1 g (66 % yield) of ethyl 1-(5,6-dimethyl-1*H*-benzo[*d*]imidazol-2-yl)piperidine-4-carboxylate.
(b) Preparation of the intermediary compound 1-(5,6-dimethyl-1*H*-benzo[*d*]imidazol-2-yl)piperidine-4-carboxylic acid: 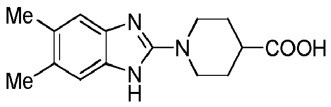 Ethyl 1-(5,6-dimethyl-1*H*-benzo[*d*]imidazol-2-yl)piperidine-4-carboxylate (1 g, 3.3 mmol), sodium hydroxide (16.5 mmol) and a mixture of ethanol and water (4:1) were stirred for 30-45 minutes at room temperature. The reaction mixture was poured out on ice, the organic phase evaporated and the aqueous phase washed with ethyl acetate. The aqueous phase was neutralized with aqueous hydrochloric acid (1.5 N), the formed precipitate filtered off and dried *in vacuo.* The residue was recrystallized from petroleum ether (50 mL) to give 520 mg (57 % yield) of 1-(5,6-dimethyl-1*H*-benzo[*d*]imidazol-2-yl)piperidine-4-carboxylic acid. ¹H NMR (300.13 MHz, DMSO-d₆) δ 11.07 (broad), 6.95 (s, 2H), 3.98 (d, 2H), 2.99 (triplett, 2H), 2.46 (m, 1H) 2.22 (s, 6H), 1.88 (d, 2H), 1.57 (m, 2H).
(c) Preparation of the intermediate compound (R)-1-(5,6-dimethyl-1H-benzo[d]imidazol-2-yl)-N-(tetrahydrofuran-3-yl)piperidine-4-carboxamide: 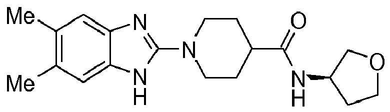 1-(5,6-Dimethyl-1H-benzo[d]imidazol-2-yl)piperidine-4-carboxylic acid (1.0 g, 3.6 mmol), (R)-(+)-tetrahydrofuran-3-amine 4-methylbenzenesulfonate (4.3 mmol), 2-(7-aza-1H-benzotriazole-1-yl)-1,1,3,3-tetramethyluronium hexafluorophosphate (HATU, 7.2 mmol), *N,N-*diisopropylethylamine (Hünig's base, DIEA, 7.2 mmol) and N,N-dimethylformamide (25 mL) was stirred at room temperature for 1 hour. After the appropriate work-up, the residue was recrystallized from dichloromethane/petroleum ether to give 0.96 g (77 % yield) of (R)-1-(5,6-dimethyl-1H-benzo[d]imidazol-2-yl)-N-(tetrahydrofuran-3-yl)piperidine-4-carboxamide.
(d) A mixture of (R)-1-(5,6-dimethyl-1H-benzo[d]imidazol-2-yl)-N-(tetrahydrofuran-3-yl)piperidine-4-carboxamide (120 mg, 0.35 mmol), dimethylsulfoxide (6 mL), caesium carbonate (1.4 mmol), 6-bromoquinoline (1.4 mmol), 8-hydroxyquinoline (0.07 mmol), polyethylene glycol (0.89 g) and copper(I) oxide (0.0175 mmol) was subjected to microwave conditions for one hour at 120°C. The reaction mixture was filtered and subjected to preparative hplc (performed on a Gilson-Finnigan ThermoQuest AQA system equipped with a Zorbax SB-C8 (5 µm, 21.2 x 150 mm) column, using methanol/water (0.05 % formic acid) gradients at a flow rate of 15 mL/min with UV (214 or 254 nm) and MS (ESI) detection) to give 15 mg (9 % yield) of (R)-1-(5,6-dimethyl-1-(quinolin-6-yl)-1H-benzo[d]imidazol-2-yl)-N-(tetrahydrofuran-3-yl)piperidine-4-carboxamide as an off-white solid. LC-MS (m/z) 470.1 (M+1).

### Comparative Example 16: (R)-1-(5,6-dichloro-1-(pyridin-2-yl)-1H-benzo[d]imidazol-2-yl)-N-(tetrahydrofuran-3-yl)piperidine-4-carboxamide:

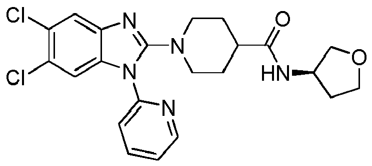

A mixture of (R)-1-(5,6-dichloro-1H-benzo[d]imidazol-2-yl)-N-(tetrahydrofuran-3-yl)-piperidine-4-carboxamide (100 mg, 0.261 mmol) dimethylsulfoxide (2 mL), caesium carbonate (0.392 mmol), 2-iodopyridine (0.783 mmol), 8-hydroxyquinoline (0.104 mmol), polyethylene glycol (0.511 mmol) and copper(I) oxide (0.0522 mmol) was subjected to microwave conditions for two hour at 120°C. The reaction mixture was filtered and subjected to preparative hplc (performed on a Gilson-Finnigan ThermoQuest AQA system equipped with a Zorbax SB-C8 (5 µm, 21.2 x 150 mm) column, using methanol/water (0.05 % formic acid) gradients at a flow rate of 15 mL/min with UV (214 or 254 nm) and MS (ESI) detection) to give 30 mg (25 % yield) of (R)-1-(5,6-dichloro-1-(pyridin-2-yl)-1H-benzo[d]imidazol-2-yl)-N-(tetrahydrofuran-3-yl)piperidine-4-carboxamide as a pale yellow solid. LC-MS (m/z) 460.3 (M+1).

### BIOLOGICAL TESTS

Prostaglandin detection kits were purchased from Cayman Chemicals and used according to the instruction of the manufacturer.

### Thiobarbituric acid assay (TBA-MDA assay or Malondialdehyde assay)

Malondialdehyde is a product of lipid peroxidation and reacts with thiobarbituric acid forming a red product that absorbs light at 535 nm (W.G. Niehaus, Jr and B. Samuelsson, Eur. J. Biochem 6, 126 (1968) and is also fluorescent. The extinction coefficient of the TBA-MDA conjugate is 1.56 x 10⁵ M⁻¹ cm⁻¹ (E.D. Wills. Biochem. J. 113, 315 (1969). The method used for detection of inhibition of mPGEs⁻¹ is based on the detection of the amount of remaining PGH₂. This method was described more than 20 years ago by Basevich et al (Bioorg. Khim. 1983, 9(5), 658-665).

The assay used was a modified variant and used citric acid instead of the TCA-TBA-HCl reagent described in the original assay. In this assay, recombinant membrane-bound human mPGEs-1 was incubated with PGH₂. The reaction was stopped by adding citric acid to a final pH of 3 and a large excess of iron(II) chloride (20 mM) to convert any remaining PGH₂ into MDA and 12-HHT. TBA reagent was finally added (0.67 %) and the samples were heated at 80°C for 30 min. The fluorescence of the conjugate was measured at 485/545 nm.

The product of mPGEs-1 (PGE2) was not measured directly in this assay, but rather the remaining substrate (PGH₂) indirectly by adding FeCl₂ that converts PGH2 into MDA and 12-HHT. As a positive control, a known mPGEs-1 inhibitor, MK-886, was used and the new inhibitors were compared with the inhibition of MK-886.

The arithmetic mean values from the human mPGEs-1 TBA-MDA assay (IC₅₀ in µM) are shown in Table 1.

### A549 cell-assay

A549 lung carcinoma cells, seeded at a density of 10, 000 cells/well, were grown in 96 well tissue culture plates. TNFalfa (5 ng/mL) and IL-1beta (5 ng/mL) were added and the cells were incubated for 16 hours. Cells were washed in PBS and test compounds at the appropriate concentration in HBSS/0.1% BSA were added. After 30 minutes of incubation with test compounds, 10 µM arachidonic acid was added and cells were further incubated for 30 minutes. Supernatant was collected and analyzed for PGE₂ content by EIA according to manufacturer's instructions. The arithmetic mean values from the A549 cell-assay are shown in Table 1.

**Table 1. Inhibition of human mPGEs-1 and cellular PGE2 synthesis.**

| **Example** | **Human mPGEs-1** | **A549 cell-assay** |
|---|---|---|
| | **IC₅₀ (µM)** | **IC₅₀ (µM)** |
| **1** | 0.020 | 0.043 |
| **2** | 0.025 | 0.058 |
| **3** | 0.037 | 0.058 |
| **4** | 0.035 | 0.044 |
| **5** | 0.034 | 0.004 |
| **6** | 0.058 | 0.05 |
| **7** | 0.032 | 0.03 |
| **8** | 0.031 | 0.04 |
| **9** | 0.051 | 0.067 |
| **10** | 0.042 | 0.061 |
| **11** | 0.047 | 0.016 |
| **12** | 0.026 | 0.024 |
| **13** | 0.03 | n.t. |
| **14** | 0.021 | n.t. |
| **15** | 0.065 | 0.066 |
| **16** | 0.15 | n.t. |

| | | |
|---|---|---|
| t. = not tested | | |

### Conclusions from the biological tests

1) The Examples of the present invention are all capable of inhibiting human mPGEs-1. Moreover, compounds tested in the A549 cell-assay showed inhibition of the production of PGE₂.
2) All Examples 1-14 of the present invention inhibit human mPGEs-1 more effectively than Examples 15 and 16.
3) All Examples 1-14, except Example 9, of the present invention showed increased inhibition of the production of PGE₂, compared to Examples 15 and 16.

## Claims

1. A compound of formula (I) 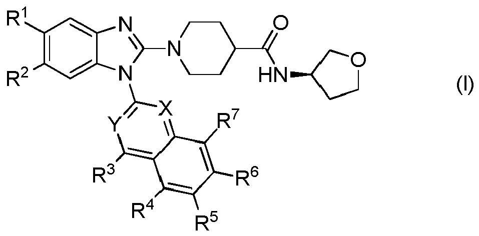 wherein
R¹ and R² are each independently selected from methyl and chlorine;
R³, R⁴, R⁵, R⁶ and R⁷ are each independently selected from hydrogen, fluorine, chlorine, and C₁-C₄ alkyl;
X is selected from -N- and -CH-;
Y is seleceted from -N- and -CH-;
provided that X and Y are different,
or a pharmaceutically acceptable salt thereof.

2. A compound according to claim 1, wherein R³, R⁴, R⁵, R⁶ and R⁷ are each independently selected from hydrogen, fluorine, chlorine, and methyl; or a pharmaceutically acceptable salt thereof.

3. A compound according to claim 1 and 2, wherein R⁴ and R⁶ are hydrogen; or a pharmaceutically acceptable salt thereof.

4. A compound according to any one of the claims 1-3, wherein R⁴, R⁶, and R⁷ are hydrogen; or a pharmaceutically acceptable salt thereof.

5. A compound according to any one of the claims 1-4, wherein X is -N- and Y is -CH-; or a pharmaceutically acceptable salt thereof.

6. A compound according to any one of the claims 1-5, wherein R¹ is chlorine; or a pharmaceutically acceptable salt thereof.

7. A compound according to any one of the claims 1-5, wherein R¹ and R² are both chlorine; or a pharmaceutically acceptable salt thereof.

8. A compound according to claim 1, selected from:
(*R*)-1-(5,6-dichloro-1-(quinolin-2-yl)-1*H*-benzo[*d*]imidazol-2-yl)-N-(tetrahydrofuran-3-yl)piperidine-4-carboxamide;
(R)-1-(5-chloro-6-methyl-1-(quinolin-2-yl)-1H-benzo[d]imidazol-2-yl)-N-(tetrahydrofuran-3-yl)piperidine-4-carboxamide;
(*R*)-1-(6-chloro-5-methyl-1-(quinolin-2-yl)-1*H*-benzo[*d*]imidazol-2-yl)-*N*-(tetrahydrofuran-3-yl)piperidine-4-carboxamide;
(*R*)-1-(5,6-dichloro-1-(6-chloroquinolin-2-yl)-1*H*-benzo[d]imidazol-2-yl)-*N*-(tetrahydrofuran-3-yl)piperidine-4-carboxamide;
(*R*)-1-(1-(6-chloroquinolin-2-yl)-5,6-dimethyl-1*H*-benzo[*d*]imidazol-2-yl)-*N*-(tetrahydrofuran-3-yl)piperidine-4-carboxamide;
(*R*)-1-(5,6-dichloro-1-(4-methylquinolin-2-yl)-1*H*-benzo[*d*]imidazol-2-yl)-*N*-(tetrahydrofuran-3-yl)piperidine-4-carboxamide;
(*R*)-1-(5-chloro-1-(6-chloroquinolin-2-yl)-6-methyl-1*H*-benzo[d]imidazol-2-yl)-*N-*(tetrahydrofuran-3-yl)piperidine-4-carboxamide;
(*R*)-1-(6-chloro-1-(6-chloroquinolin-2-yl)-5-methyl-1*H*-benzo[*d*]imidazol-2-yl)-*N-*(tetrahydrofuran-3-yl)piperidine-4-carboxamide;
(*R*)-1-(5,6-dichloro-1-(6-fluoroquinolin-2-yl)-1*H*-benzo[*d*]imidazol-2-yl)-*N*-(tetrahydrofuran-3-yl)piperidine-4-carboxamide;
(*R*)-1-(5-chloro-1-(6-fluoroquinolin-2-yl)-6-methyl-1*H*-benzo[*d*]imidazol-2-yl)-*N-*(tetrahydrofuran-3-yl)piperidine-4-carboxamide;
(*R*)-1-(6-chloro-1-(6-fluoroquinolin-2-yl)-5-methyl-1*H*-benzo[d]imidazol-2-yl)-*N-*(tetrahydrofuran-3-yl)piperidine-4-carboxamide;
(*R*)-1-(5,6-dichloro-1-(8-fluoroquinolin-2-yl)-1*H*-benzo[d]imidazol-2-yl)-*N*-(tetrahydrofuran-3-yl)piperidine-4-carboxamide; and
(R)-1-(5,6-dichloro-1-(isoquinolin-3-yl)-1H-benzo[d]imidazol-2-yl)-N-(tetrahydrofuran-3-yl)piperidine-4-carboxamide;
or a pharmaceutically acceptable salt thereof.

9. A compound according to any one of the claims 1 to 8, or a pharmaceutically acceptable salt thereof, for use as a medicament.

10. A pharmaceutical composition comprising a therapeutically effective amount of a compound according to any one of the claims 1 to 8, or a pharmaceutically acceptable salt thereof, in admixture with at least one pharmaceutically acceptable excipient.

11. A compound according to any one of the claims 1 to 8, or a pharmaceutically acceptable salt thereof, for use in the treatment and/or prevention of a disorder selected from
an inflammatory disease, said disease preferably being selected from rheumatoid arthritis and other inflammatory diseases related to arthritis such as ankylosing spondylitis, inflammatory bowel disease, inflammation-related nephrototoxicity osteoarthritis, periodontitis, dermatitis including psoriasis, eczema, swelling and (intra-)ocular inflammation;
pain, said pain preferably being selected from inflammatory pain, post-operative pain, fibromyalgia, dysmenorrhea, migraine, arthrotic pain, arthritic pain, pain in connection with osteoarthritis, endometriosis, cephalalgia, pain due to gall-stones, pain due to kidney stones, pain due to gout, neuropathic pain and pain due to metastasis;
an autoimmune disease, said autoimmune disease preferably being selected from rheumatoid arthritis, multiple sclerosis and Kawasaki disease;
a breathing disorder, said disorder preferably being selected from apnea or other respiratory distress symptoms in adults and children, sudden infant death syndrome (SIDS), asthma and other chronic obstructive lung-diseases and sarcoidosis;
cancer, said cancer preferably being selected from colorectal cancer, breast cancer, gastric tumorigenesis, intestinal tumorogenesis, bone metastatic cancer, lung cancer, oesophageal adenocarcinoma, head and neck squamous cell carcinoma, papillary thyroid carcinoma, gliomas and pancreatic, cervical and prostate cancers, epithelial ovarian cancer and pancreas cancer;
a cardiovascular disease due to atherosclerosis, such as myocardial infarction and stroke;
a neurodegenerative disease, said disorder preferably being selected from Alzheimer's disease, amyotrophic lateral sclerosis, memory impairment and seizure-induced neuronal damage;
a bone disease, said disorder preferably being selected from bone loss and pachydermoperiostosis; and
fever related-anorexia, inflammation related-anorexia, overactive bladder or familial adenomatous polyposis (FAP) condition.

12. A combination product comprising: (A) a compound according to any one of the claims 1 to 8, or a pharmaceutically-acceptable salt thereof, and (B) another therapeutic agent, wherein each of components (A) and (B) is formulated in admixture with a pharmaceutically-acceptable excipient.

13. A combination product as claimed in claim 12, wherein the other therapeutic agent is an agent useful in the treatment of inflammation.

14. A combination product as claimed in claim 12 or claim 13, which comprises a kit of parts comprising components: (A) a pharmaceutical formulation including a compound as defined above, or a pharmaceutically-acceptable salt thereof, in admixture with a pharmaceutically-acceptable excipient; and (B) a pharmaceutical formulation including another therapeutic agent in admixture with a pharmaceutically acceptable excipient, which components (A) and (B) are each provided in a form that is suitable for administration in conjunction with the other.

15. A method of treatment of a disorder selected from an inflammatory disease, pain, an autoimmune disease, a breathing disorder, cancer, a cardiovascular disease due to atherosclerosis, a neurodegenerative disease, a bone disease, fever or inflammation-related anorexia, familial adenomatous polyposis (FAP) condition or overactive bladder, which method comprises administration of a therapeutically effective amount of a compound according to any one of the claims 1 to 8, or a pharmaceutically-acceptable salt thereof, to a mammal subject suffering from, or susceptible to, such a disorder.
